# EUROPEAN PATENT APPLICATION

(11) **EP 3 964 221 A1**
(43) Date of publication of application: **09.03.2022**
(21) Application number: 20194880.9
(22) Date of filing: 07.09.2020
(51) Int. Cl.: A61K 36/61, A61K 36/752, A61P 31/14, A61P 11/00, A61P 31/16

(54) **ANTIVIRAL AGENTS**

(71) Applicant: G. Pohl-Boskamp GmbH & Co. KG, 25551 Hohenlockstedt (DE)
(72) Inventor: Ueck, Henning, 25524 Beckmünde (DE); Röschmann-Doose, Kristina, 24106 Kiel (DE); Thomsen, Jörn, 24220 Flintbek (DE); WITTIG, Thomas Heinz, 25551 Lohbarbek (DE)
(74) Representative: Lahrtz, Fritz

(57) **Abstract**

The present invention relates to a composition comprising eucalyptus oil and sweet orange oil for use in a method for the treatment of a viral infection.

## Description

Viruses are involved in the aetiology of a large number of diseases and infections of the respiratory tract are among the most widespread. The common cold and its two manifestations, acute rhinosinusitis and bronchitis are globally occurring diseases of mainly viral aetiology which do not result from infection by one specific but several potential viruses (Fokkens et al., Executive summary of EPOS 2020 including integrated care pathways. Rhinology. 2020 Apr 1;58(2):82-111). The most important pathogens are Rhinoviruses which itself are differentiated into three species (A-C) each with multiple types resulting in about 150 known different genotypes (Palmenberg and Gern, 2015). Further pathogens encompass viruses from several families like Respiratory Syncytial Virus, Influenza, Adenovirus, Coronavirus and Parainfluenza which can all be detected in nasal washes of patients with diseases of the upper respiratory tract (Mäkelä et al., 1998, DeMuri et al., 2016). The sensitivity to medical treatment differs between the different viruses and even between individual types of rhinoviruses (Glatthaar-Saalmüller et al., 2017). The viruses are easily spread among humans by hand-to-hand contact or via air-borne droplet infection with pronounced peaks of incidences of common colds in late autumn and late winter. Although the disease is self-limiting, patients suffer from symptoms like nasal blockage, facial pain, cough and/or sore throat which last for several days whereby severity correlates well with viral load (Arruda et al., 1997, Bagga et al., 2013, Jacobs et al., 2013). As a result, the common cold is responsible for a significant part of sick leaves and therefore exerts a substantial economic burden to the healthcare system (Ehlken et al., 2015).

Apart from causing the common cold, viral infections are also the cause of several viral diseases which represent a major threat for humanity. Not only the SARS-Cov-2 pandemic which started in fall 2019, but also the regular influenza pandemics result in serious health care and economic problems. Despite the intensive scientific research in the field of antiviral agents, it is still today very difficult to control viral diseases, and for many viruses including SARS-Cov-2, no real treatment options or efficient vaccines are currently available. Especially, there are only few antiviral agents available that inhibit the replication of various viruses. Furthermore, many antiviral agents result in significant side effects.

Therefore, there is a continuous need for efficient antiviral agents.

The present invention relates to a composition for use in a method for the treatment of a viral infection comprising
a) at least one essential oil chosen from the group of eucalyptus oil and myrtle oil, and
b) at least one essential oil of a citrus fruit.

The citrus fruit may be *Citrus medica, Citrus bergamia, Citrus* × *aurantium, Citrus* × *sinensis, Citrus medica var. sarcodactylis* × *Citrofortunella mitis, Citrus reticulata* × *maxima, Citrus myrtifolia* , *Citrus medica, Citrus reticulate, Citrus medica, Citrus glauca, Citrus medica, Citrus australasica, Citrus medica, Citrus* × *paradise, Citrus medica, Citrus tamurana, Citrus* × *iyo, Citrus sphaerocarpa, Citrus hystrix, Citrus aurantiifolia, Citrus nobilis* × *Citrus deliciosa, Citrus unshiu* × *Citrus sinensis, Citrus japonica, Citrus limon, Citrus reticulate, Citrus mangshanensis, Citrus* × *meyeri, Citrus medica, Citrus myrtifolia, Citrus* × *sinensis, Citrus grandis* × C. *Paradisi*/*Citrus maxima*/*Citrus grandis, Citrus* × *latifolia, Citrus maxima* or *Citrus grandis, Citrus maxima* × *medica, Citrus* × *limonia, Citrus australis, Citrus unshiu, Citrus cavaleriei* × *C. maxima, Citrus flaviculpus hort. ex Tanaka (Ogonkan)* × *Citrus unshiu, Citrus sudachi, Citrus limetta, Citrus* × *depressa, C. reticulata* × C. *maxima* or xC. *paradise, Citrus tangerine, C. reticulata* × C. *sinensis, Citrus reticulata* × *Citrus paradise, Citrus cavaleriei* × *C. reticulate,* preferably *citrus reticulata* (mandarin, clementine), *citrus limon* (lemon), *citrus aurantifolia, citrus x* paradise (grapefruit) or *citrus x sinensis* (orange)

In a preferred embodiment, the composition comprises eucalyptus oil and sweet orange oil.

In an even more preferred embodiment, the composition comprises eucalyptus oil, sweet orange oil, myrtle oil and lemon oil.

All these oils are essential oils.

The terms "viral infection" as used herein refers to the invasion of an organism's tissues or cells by viruses and related agents such as viroids, their multiplication and reaction with host cells, as well as the resulting conditions and diseases.

The eucalyptus oil is preferably used in the form of a rectified essential oil, which preferably contains more than 70 percent of 1,8-cineol, as well as [alpha]-pinene, preferably (+)-[alpha]-pinene.

In the composition of the invention, the share of eucalyptus oil in comparison to the sum of all essential oils may be between 20 % (w/w) and 80 % (w/w), 30 % (w/w) and 70 % (w/w), 40 % (w/w) and 70 % (w/w), between 50 % (w/w) and 70 % (w/w) or between 60 % (w/w) and 70 % (w/w). In addition, the share of eucalyptus oil may be 30 % (w/w), 40 % (w/w), 50 % (w/w), 60 % (w/w), 70 % (w/w) or 80 % (w/w). Preferably, the share of eucalyptus oil may be 61 % (w/w), 62 % (w/w), 63 % (w/w), 64 % (w/w), 65 % (w/w), 66 % (w/w), 67 % (w/w), 68 % (w/w), or 69 % (w/w). Preferably, the share of eucalyptus oil is 66 % (w/w).

The sweet orange oil used in the context of the present invention preferably contains predominantly limonene, especially (+)-limonene, that is, at least 90 percent by weight, preferably at least 93 percent by weight and particularly between 93 and 98 percent by weight of the total amount of orange oil.

In the composition of the invention, the share of sweet orange oil in comparison to the sum of all essential oils may be between 20 % (w/w) and 80 % (w/w), 30 % (w/w) and 70 % (w/w), 30 % (w/w) and 60 % (w/w), 30 % (w/w) and 50 % (w/w) or between 30 % (w/w) and 40 % (w/w). In addition, the share of sweet orange oil may be 10 % (w/w), 20 % (w/w), 30 % (w/w), 40 % (w/w), 50 % (w/w) or 60 % (w/w). Preferably, the share of sweet orange oil may be 31 % (w/w), 32 % (w/w), 33 % (w/w), 34 % (w/w), 35 % (w/w), 36 % (w/w), 37 % (w/w), 38 % (w/w), or 39 % (w/w). Preferably, the share of sweet orange oil is 32 % (w/w).

In the composition of the invention, the share of myrtle oil in comparison to the sum of all essential oils may be between 0.1 % (w/w) and 10 % (w/w), 0.1 % (w/w) and 8 % (w/w), 0.1 % (w/w) and 6 % (w/w), 0.1 % (w/w) and 3% (w/w) or between 0.1 % (w/w) and 2 % (w/w). In addition, the share of myrtle oil may be 0.1 % (w/w), 0.5 % (w/w), 0.8 % (w/w), 1 % (w/w), 1.2 % (w/w) or 1.5 % (w/w). Preferably, the share of myrtle oil may be 0.5 % (w/w), 0.6 % (w/w), 0.7 % (w/w), 0.8 % (w/w), 0.9 % (w/w), 1 % (w/w), 1.1 % (w/w), 1.2 % (w/w), or 1.3 % (w/w). Preferably, the share of myrtle oil is 1 % (w/w).

In the composition of the invention, the share of lemon oil in comparison to the sum of all essential oils may be between 0.1 % (w/w) and 10 % (w/w), 0.1 % (w/w) and 8 % (w/w), 0.1 % (w/w) and 6 % (w/w), 0.1 % (w/w) and 3% (w/w) or between 0.1 % (w/w) and 2 % (w/w). In addition, the share of lemon oil may be 0.1 % (w/w), 0.5 % (w/w), 0.8 % (w/w), 1 % (w/w), 1.2 % (w/w) or 1.5 % (w/w). Preferably, the share of lemon oil may be 0.5 % (w/w), 0.6 % (w/w), 0.7 % (w/w), 0.8 % (w/w), 0.9 % (w/w), 1 % (w/w), 1.1 % (w/w), 1.2 % (w/w), or 1.3 % (w/w). Preferably, the share of lemon oil is 1 % (w/w).

In preferred embodiment, the composition comprises 66 % (w/w) eucalyptus oil, 32 % (w/w) sweet orange oil, 1 % myrtle oil and 1 % lemon oil.

Essential oils are available from different commercial suppliers. Eucalyptus oil, sweet orange oil and lemon oil are described in the European Pharmacopoeia as pharmaceutical ingredients. Distillation and rectification are basic processing steps well known to the persons skilled in the art.

In an especially preferred embodiment of the invention, the composition is ELOM-080^{®}. ELOM-080^{®} represents the distillate of a mixture of 4 rectified essential oils: 66 % (w/w) eucalyptus, 32 % (w/w) sweet orange, 1 % myrtle and 1 % lemon. Safety and efficacy of ELOM-080^{®} have been proven in numerous GCP conform prospective, double-blinded, randomized and controlled clinical trials by superior responses in comparison to placebo and active comparators and it is recommended in guidelines for treatment of acute and chronic bronchitis and rhinosinusitis (Fokkens et al., 2012, Stuck et al., 2017, Kardos et al., 2019). The preclinical record already documented the secretolytic, mucolytic, secretomotoric, antiinflammatory, antioxidative, antimicrobial and bronchospasmolytic effects of ELOM-080^{®} (Kaschke et al., 1997, Beuscher et al., 1997, Beuscher et al., 1998, App, 2000, Graßmann et al., 2000, Rantzsch et al., 2009, Begrow et al., 2012).

ELOM-080^{®} is a trade mark of Desmoid Aktiengesellschaft, 20354 Hamburg, DE. A medicinal product containing ELOM-80^{®} is sold as Gelomyrtol^{®} by Pohl Boskamp GmbH & Co. KG.

In a further aspect, the invention further relates to the use of a composition comprising eucalyptus oil, sweet orange oil, myrtle oil and lemon oil as an antiviral agent. All embodiments disclosed above also relate to this aspect of the invention.

In the example section, it has been shown that the composition used in the context of the present invention has antiviral effects against a broad range of viruses. Consequently, it may be used in the treatment of a viral infection caused by every virus and may be selected from the group consisting of enveloped viruses or non-enveloped viruses. In the context of the present invention, an enveloped virus is a virus with a lipoprotein envelope surrounding the nucleoprotein core of the virus.

In a preferred embodiment, the virus is an enveloped virus. The virus may be selected from the group consisting of orthomyxoviruses, preferably influenza viruses, preferably influenza A virus, paramyxoviruses, preferably measles virus, mumps virus, or RSV (Respiratory Syncytial Virus), parainfluenzaviruses, coronaviruses, togaviruses, rubella virus, rhabdoviruses, rabies virus, retroviruses, preferably HIV (Human Immunodeficiency Virus) or human T-Cell lymphotropic virus.

In an even more preferred embodiment, the virus is an enveloped RNA virus. As shown in Example 1, the composition used in the context of the present invention has been shown to be especially active against enveloped RNA viruses.

Preferably, the virus is selected from the group consisting of influenza viruses, RSV, coronaviruses, HIV, and parainfluenza virus.

Preferably, the virus is a coronavirus. Coronaviruses (CoVs) (order *Nidovirales*, family *Coronaviridae*, subfamily *Coronavirinae*) are enveloped viruses with a positive sense, single-stranded RNA genome. With genome sizes ranging from 26 to 32 kilobases (kb) in length, CoVs have the largest genomes for RNA viruses. Based on genetic and antigenic criteria, CoVs have been organised into three groups: α-CoVs, β-CoVs, and γ-CoVs. Coronaviruses primarily infect birds and mammals, causing a variety of lethal diseases that particularly impact the farming industry They can also infect humans and cause disease to varying degrees, from upper respiratory tract infections (URTIs) resembling the common cold, to lower respiratory tract infections (LRTIs) such as bronchitis, pneumonia, and even severe acute respiratory syndrome (SARS). In recent years, it has become increasingly evident that human CoVs (HCoVs) are implicated in both URTIs and LRTIs, validating the importance of coronaviral research as agents of severe respiratory illnesses.

Preferably, the coronavirus is SARS-CoV, SARS-CoV-2 or MERS-CoV. Most preferably, the corona virus is SARS-CoV-2.

SARS-CoV-2 is a positive-sense single-stranded RNA (+ssRNA) virus which belongs to the beta-coronavirus family and shares substantial genetic and functional similarity with other pathogenic human beta-coronaviruses, including Severe Acute Respiratory Syndrome Coronavirus ("SARS-CoV", also called SARS-CoV-1) and Middle East Respiratory Syndrome Coronavirus ("MERS-CoV").

Alternatively, the virus may also be a non-enveloped virus, and may be a rhinovirus.

The composition used according to present invention may be administered prophylactically before viral infection to inhibit viral infection after exposure to virus. Alternatively or additionally, it may be administered after viral infection to inhibit or reduce viral replication and production of infectious progeny.

In a preferred embodiment, the composition is administered during an ongoing viral infection to treat the infection.

The composition may be administered subcutaneously, intramuscularly, intravenously, topically and preferably orally. In a preferred embodiment, the composition is administered orally in the form of soft or hard capsules or in liquid or semi-solid forms of administration. The composition may be administered in pure form or in combination with other biologically active materials, such as menthol, or in combination with conventional inactive ingredients.

The composition used in the context of the present invention may consist of the oils as defined above, or may comprise further ingredients.

Solubilizers are preferred as inactive ingredients. As such, organic solutions, preferably ethanol, surfactants, preferably sodium dodecyl sulfate or sodium desoxycholate, carbohydrates, preferably glucose or dextrose, or lipids, preferably phosphatidylcholine, as well as mixtures of these materials can be used. These solubilizers usually are employed in amounts of 0.1 to 20 percent by weight and preferably of 0.2 to 15 percent by weight, based on the amount of the oils comprised in the composition.

The composition may further comprise additional oils as solvents, preferably a natural fatty oil or a neutral oil. Preferred natural fatty oils are rapeseed oil, coconut oil and corn oil. Preferred neutral oils are medium chain length (C6-C12) triglycerides, especially caprylic and capric triglycerides, such as medium chain triglycerides according to the European Pharmacopoeia.

In accordance with the invention, the weight ratio of eucalyptus oil and sweet orange oil as defined above in the composition may be between 1:10 and 10:1, with a preferred ratio being 2:1. The weight content of eucalyptus oil and sweet orange oil, respectively, as defined above may be from 1-80%, 10-75%, or 40-70%.
In the case of hard or soft capsules, for example, the content of the oils as defined above preferably ranges from 1 to 80 percent by weight, especially from 20 to 50 percent by weight and particularly from 30 to 40 percent by weight. One capsule preferably contains 40 to 350 mg and particularly 100 to 300 mg of the composition of the invention, preferably 300 mg. The capsule may also contain 400 to 700 mg of the composition of the invention, preferably 600 mg.

The daily dose may be between 0.70 g to 4.8 g, preferably from 0.72 g to 4.8 g. Preferred daily dosages are 1.2 g (e.g. administered in 4 capsules a 300 mg) or 2.4 g (e.g. administered in 4 capsules a 600 mg). The administration may 1-4 times daily, preferably 2-4 or 3-4 times daily. The duration of the treatment may be 2-10 days, or one, two or three weeks.

In a preferred embodiment, the viral infection causes hypoxemia or an acute respiratory distress syndrome. Such effects are often seen in the context of respiratory diseases caused by viruses.

Hypoxemia refers to the low level of oxygen in blood, and the more general term hypoxia is an abnormally low oxygen content in any tissue or organ, or the body as a whole. Hypoxemia can cause hypoxia (hypoxemic hypoxia), but hypoxia can also occur via other mechanisms, such as anemia.

In a preferred embodiment, the composition for use according to the present invention includes the treatment of a viral disease caused by the viral infection.

The viral disease may be any disease caused by the virus as defined above. In a preferred embodiment, the viral disease is an acute or chronic viral respiratory disease, preferably a viral pneumonia, a viral sinusitis and rhinosinusitis or a viral bronchitis.

Preferably, the viral respiratory disease is COVID-19. This disease is provoked by SARS-CoV-2.

The invention is further explained by the following examples and figures, which are intended to illustrate, but not to limit the present invention.

The invention further relates to a composition as defined above for us in a method for treatment hypoxemia. All embodiments disclosed above with respect to the composition to be used for the treatment of a viral infection according to the present invention also apply to this aspect of the invention. The term "hypoxia" is also defined above.

In a preferred embodiment, the hypoxemia to be treated in the context of the present invention is an accompanying phenomenon of a respiratory disease. The respiratory disease may be any disease of the respiratory tract and is preferably a viral or bacterial respiratory disease.

In a more preferred embodiment, the respiratory disease is a viral respiratory disease as defined above.

### Short Description of the Figures

Fig. 1: Cytotoxicity of ELOM-080^{®} on MDCK, HeLa and Hep-2 cell cultures on day 1 (black), day 2 (dark grey) and day 3 (light grey) in the MTT assay. Numbers correspond to ELOM-080^{®} concentrations (µg mL⁻¹) tested in the assay.
Fig. 2: Dose response curve of the preventive (=virucidal) antiviral activity of ELOM-080^{®} against Flu A (A), RV (B), RSV (C) and AV (D) expressed as mean inhibition in % plotted against concentration of test substance during pretreatment of viruses.
Fig. 3: Representative visual examples of the effects of ELOM-080^{®} treatment on FluA infectivity in plaque reduction assay. Numbers correspond to ELOM-080^{®} concentrations tested in the assay. EtOH: solvent control, MEM: medium control, Ribavirin: active control.
Fig. 4: Dose response curve of the curative antiviral activity of ELOM-080^{®} against Flu A (A), RV (B), RSV (C) and AV (D) expressed as inhibition in % plotted against concentration of test substance in the cell culture during plaque reduction (A-C) or CPE assay (D), mean ± standard deviation.

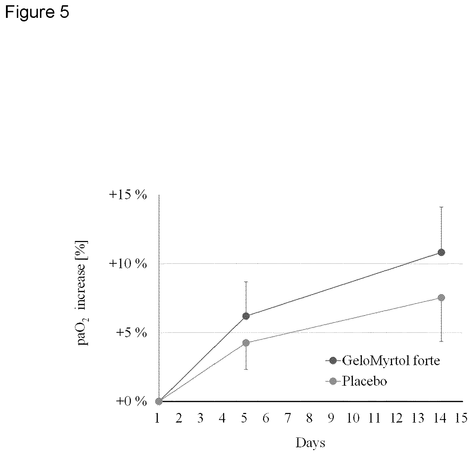

Fig. 5: Relative increase of partial pressure of oxygen (PaO2) compared to baseline in a 14 days treatment course with 4 x 300 mg ELOM-080^{®} forte or placebo (Mean ± SEM)

### Examples

### Example 1

### Antiviral activity of ELOM-080^{®} against respiratory pathogens in in-vitro assays.

This example demonstrates that the present inventors could surprisingly show that a composition comprising eucalyptus oil and sweet orange oil as the major active ingredients has anti-viral properties and, therefore, is suitable in the treatment of viral infections.

Viruses are involved in the aetiology of a large number of diseases and infections of the respiratory tract are among the most widespread. The common cold and its two manifestations, acute rhinosinusitis and bronchitis are globally occurring diseases of viral aetiology which do not result from infection by one specific but several potential viruses (Mäkelä et al., 1998). The most important pathogens are Rhinoviruses which itself are differentiated into three species (A-C) each with multiple types resulting in about 150 known different genotypes (Palmenberg and Gern, 2015). Further pathogens encompass viruses from several families like Respiratory Syncytial Virus, Influenza, Adenovirus, Coronavirus and Parainfluenza which can all be detected in nasal washes of patients with diseases of the upper respiratory tract (Mäkelä et al., 1998, DeMuri et al., 2016). The sensitivity to medical treatment differs between the different viruses and even between individual types of rhinoviruses (Glatthaar-Saalmüller et al., 2017). The viruses are easily spread among humans by hand-to-hand contact or via air-borne droplet infection with pronounced peaks of incidences of common colds in late autumn and late winter. Although the disease is self-limiting, patients suffer from symptoms like nasal blockage, facial pain, cough and/or sore throat which last for several days whereby severity correlates well with viral load (Arruda et al., 1997, Bagga et al., 2013, Jacobs et al., 2013). As a result, the common cold is responsible for a significant part of sick leaves and therefore exerts a substantial economic burden to the healthcare system (Ehlken et al., 2015).

Although only in few cases of acute rhinosinusitis and bronchitis bacteria occur in secondary superinfection, treatment of patients with antibiotics in early phase of disease is still common in clinical practice. However, the clinical benefit is questionable (Smith et al., 2017, Lemiengre et al., 2018). Moreover premature discontinuation of antibiotic treatment may lead to development of resistances among bacterial pathogens (Hansen et al., 2015). In contrast, herbal medicinal products facilitate relieve of symptoms and recovery with at the same time low risk of detrimental effects which results in a favourable risk-benefit ratio.

ELOM-080^{®} represents the distillate of a mixture of 4 rectified essential oils: eucalyptus, sweet orange, myrtle and lemon. The major monoterpenes 1,8-cineole, (+)-limonene and (+)-α-pinene serve as markers *in vivo* and *in vitro,* but it is unlikely they encompass the full pharmacological spectrum. Safety and efficacy of ELOM-080^{®} have been proven in numerous GCP conform prospective, double-blinded, randomized and controlled clinical trials by superior responses in comparison to placebo and active comparators and it is recommended in guidelines for treatment of acute and chronic bronchitis and rhinosinusitis (Fokkens et al., 2012, Stuck et al., 2017, Kardos et al., 2019). The preclinical record already documented the secretolytic, mucolytic, secretomotoric, anti-inflammatory, antioxidative, antimicrobial and bronchospasmolytic effects of ELOM-080^{®} (Kaschke et al., 1997, Beuscher et al., 1997, Beuscher et al., 1998, App, 2000, Graßmann et al., 2000, Rantzsch et al., 2009, Begrow et al., 2012). This study was performed in order to test whether ELOM-080^{®} also exerts *in-vitro* activity against viral pathogens involved in the aetiology of diseases of the respiratory tract.

For experiments, ELOM-080^{®} was diluted 1:5 in 96 % EtOH in order to obtain stock solutions which were further diluted with cell culture medium (MEM) leading to final drug concentrations of 0.9 - 2670 µg mL⁻¹ in the assays.

The virostatic ribavirin Virazol^{®} (abcam Biochemicals) at concentrations between 2.5 and 50 µg mL⁻¹ and a plant-derived substance (trade name 'Sinupret Trockenextrakt', 10 µg mL⁻¹) were included as reference drugs in order to validate the test system.

Influenza A virus (Flu A, H1N1), Respiratory Syncytial Virus (RSV, type long strain) and Adenovirus C (AV, subtype 5) were obtained from the Interfaculty Institute of Microbiology and Infection Medicine at the University of Tübingen, Germany. Rhinovirus (RV, subtype 14) was obtained from the Institute of Virology at the Friedrich-Schiller - University, Jena, Germany.

For *in-vitro* testing, adequate cell cultures systems were applied. Madin-Darby canine kidney (MDCK) cells and serum-free minimum essential medium (MEM) supplemented with 1 µg mL⁻¹ of trypsin (Sigma-Aldrich), 2 mmol L⁻¹ of L-glutamine (PAN-Biotech), 100 U mL⁻¹ of penicillin (PAN-Biotech) and 0.1 mg mL⁻¹ of streptomycin (PAN-Biotech) were used for FluA. RV was grown on HeLa cells and AV and RSV were propagated on HEp-2 cells, both cell lines were cultured in MEM containing 2% (v/v) foetal calf serum (PAN-Biotech), 2 mmol L⁻¹ of L-glutamine (PAN-Biotech) 100 U mL⁻¹ of penicillin (PAN-Biotech) and 0.1 mg mL⁻¹ of streptomycin (PAN-Biotech).

### Cytotoxicity of ELOM-080^{®}:

In order to determine physiological ELOM-080^{®} concentrations for the antiviral activity assays, IC₅₀ of non-infected and infected cells were assessed in cytotoxicity tests using the MTT assay over three days (Mosmann, 1983). The test quantifies mitochondrial enzyme activity which directly correlates with cell viability. Furthermore, cell monolayers were assessed microscopically based on the criteria cell morphology, vacuolization and destruction of cell monolayer. Cells were grown in dilutions of ELOM-080^{®} between 0.2 and 4 x10⁻⁴ µg mL⁻¹ and cells kept in cell culture media without any test component served as controls.

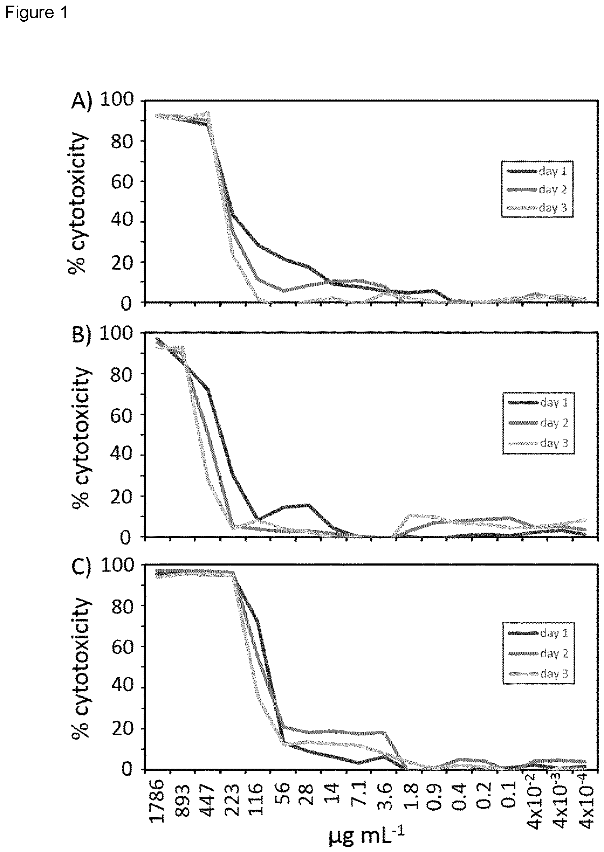

The cytotoxicity assays revealed mean IC₅₀ values for ELOM-080^{®} in non-infected MDCK, HeLa and HEp-2 of 289, 451 and 115 µg mL⁻¹, respectively (Tab. 1). No toxicity was observed for solvent controls at any tested EtOH concentration. In all cell types, toxic effects were most pronounced on the first day of the assay. On the subsequent days 2 and 3, cells initially affected recovered and returned to normal cell cycle as measured in the MTT assay (Fig. 1).

Infected cells were more sensitive to ELOM-080^{®} and consequently IC₅₀ values were lower compared to non-infected cells:

**Table 1: Results of the cytotoxicity assays of ELOM-080^{®} effects on non-infected and infected cell cultures. IC₅₀: ELOM-080^{®} concentration resulting in 50 % mortality of cells.**

| Cells | MDCK | HeLa | Hep-2 |
|---|---|---|---|
| mean IC₅₀ non-infected cells (µg mL⁻¹) | 278,9 | 451,3 | 115,2 |
| virus used for infection | FluA | RV | RSV |
| mean IC₅₀ infected cells (µg mL⁻¹) | 267,9 | 178,6 | 89,3 |

### Virucidal effects of ELOM-080^{®}:

The antiviral activity of ELOM-080^{®} was determined for FluA, RV and RSV using the plaque reduction assay and for AV applying the cytopathogenic effect (CPE) assay. This study investigated virucidal activity against cell free viruses and curative effects by application of ELOM-080^{®} 1 h after infection of cells.

In the virucidal approach, viruses were pretreated for 2h or 24 h at 4°C with varying concentration of ELOM-080^{®} (89-2673 µg mL⁻¹). Subsequently, cells were infected with a multiplicity of infection (M.O.I.) of 0.5 thereby always diluting ELOM-080^{®} to non-cytotoxic concentrations. Virus infectivity was assessed by quantifying plaque forming (PFU mL⁻¹) units after two (FluA, RV) or three (RSV) days or tissue culture infectious dose (TCID50 mL⁻¹) after Spearman Kärber for AV after five days (Kärber, 1931). Sodium dodecyl sulfate (SDS) served as positive control at concentrations corresponding to the maximally applied ELOM-080^{®} concentration. EtOH was applied as solvent control and had no virucidal effect.
In the curative approach, cell monolayers were infected with M.O.I.s of 0.0005 for FluA, RV and RSV and 0.3 or 0.1 for AV. 1 hour after infection, an agarose overlay containing ELOM-080^{®} at non-toxic concentrations between and 45 - 1786 µg mL⁻¹ was added depending on cell and virus type. Cells cultures were kept at 34° C for one (AV), two (FluA, RV) or three (RSV) days after which cells were fixed with paraformaldehyde and stained using crystal violet solution. Non-stained lesions (plaques or CPE) were quantified with the optical evaluation system EliSpot Reader (AIDSystems) in 2 to 4 replicates in three or two (AV) subsequent runs. Ribavirin against FluA, RV and RSV and the plant-derived substance (AV) served as positive controls. The solvent control with EtOH and MEM medium control had no antiviral effect.

Antiviral activity was calculated from PFU and TCID in the virucidal approach and from the number of plaques (FluA, RV, RSV) in plaque reduction assay or lesions (AV) in CPE assay in the curative approach. Antiviral activity of ELOM-080^{®} was normalized to the effect of the solvent control which was defined as 0 % inhibition. EC₅₀ values for each treatment were calculated from pooled data of replicated experiments using linear regressions in Statistica 13.

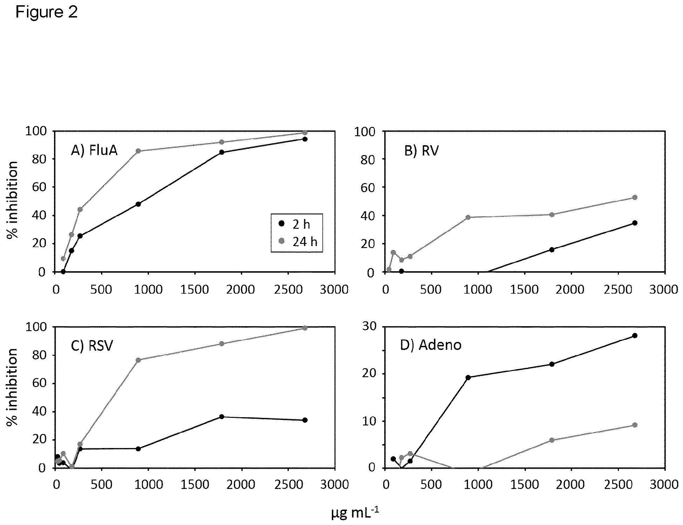

Cell-free viruses were exposed to ELOM-080^{®} concentrations up to 2679 µg mL⁻¹ for 2 or 24 h. Exposure to increasing concentrations of ELOM-080^{®} resulted in reduced infectivity of viruses which in most cases was also lowered by prolonged exposure, but sensitivity differed considerably between the 4 tested viruses (Fig. 2). Among them, FluA was found to be most sensitive to ELOM-080^{®} exposure. After 2 h pretreatment, infectivity was reduced by 50 % at 1151 µg mL⁻¹ and 24 h exposure lowered the EC₅₀ to 761 µg mL⁻¹ (Fig. 2A, Tab. 2). 50 % reduced infectivity of RV was only detected in the highest tested concentration and after 24 h exposure, whereas all other treatments resulted in an inhibition by a maximum of 40 % (Fig. 2B). RSV infectivity was inhibited by no more than 36% after 2 h exposure to any ELOM-080^{®} concentration. In contrast, 24 h treatment strongly affected RSV infectivity with almost no infectivity remaining in the highest treatment (Fig. 2C). AV responded the least sensitive to ELOM-080^{®} pretreatment. After 2 h, infectivity was reduced by up to 28 %. However, the inhibitory effect declined to less than 10 % reduction of infectivity after 24 h of incubation (Fig. 2D).
SDS applied at a concentration of 0.3% lowered infectivity of FluA, RV and RSV by 56-100 % and of AV by 52 and 63 % after 2 and 24 h incubation, respectively.

### Curative effects of ELOM-080^{®}:

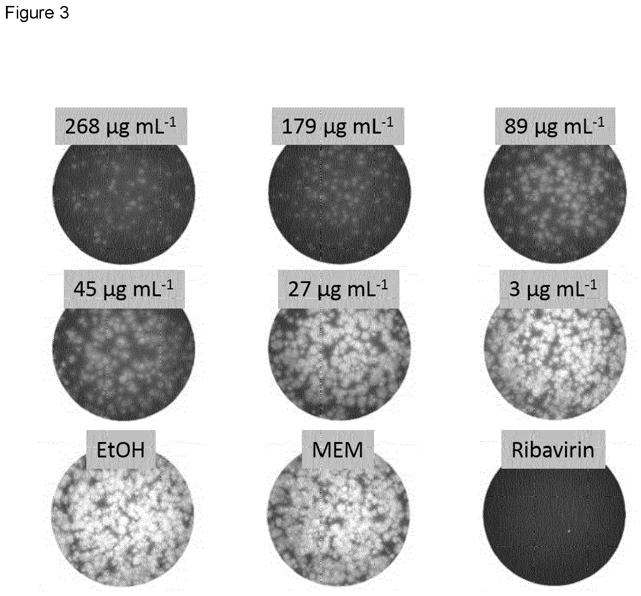

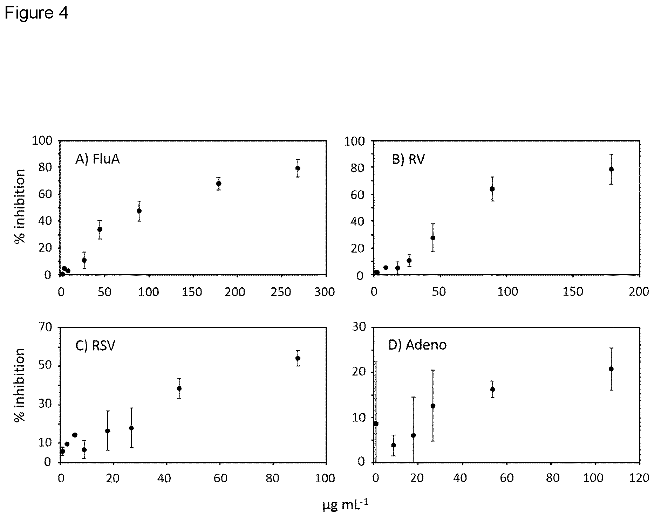

ELOM-080^{®} exhibited dose dependent antiviral effects in the curative approach when applied to cells cultures after infection. Representative examples visualize the significant reduction of plaques resulting from FluA infection in the cell monolayer when cultures were treated with increasing concentrations of ELOM-080^{®} (Fig. 3). Whereas solvent and medium controls exerted no antiviral effect, the active control Ribavirin prevented plaque formation almost completely (Fig. 3). FluA induced plaque formation was inhibited by up to 79 % with an EC₅₀ of 134 µg mL⁻¹ (Fig 4A). RV was similarly affected and inhibited by up to 78 % at an ELOM-080^{®} concentration of 179 µg mL⁻¹ (Fig. 4B). The EC₅₀ for RV was 96 µg mL⁻¹:

**Table 2: Characteristics of viruses and test systems used in this study and the EC₅₀ for ELOM-080^{®} in preventive (2h and 24 h) and curative assays. EC₅₀: ELOM-080^{®} concentration resulting in 50 % virus inhibition.**

| Virus | Influenza A | Rhinovirus | Respiratory Syncitial Virus | Adenovirus |
|---|---|---|---|---|
| type | H1N1 | subtype 14 | long strain | type 5 |
| nucleotide/envelope | RNA enveloped | RNA non-enveloped | RNA enveloped | DNA non-enveloped |
| Cells | MDCK | HeLa | HEp-2 | Hep-2 |
| 2h preventive EC₅₀ (µg mL⁻¹) | 1151 | ND | ND | ND |
| 24h preventive EC₅₀ (µg mL⁻¹) | 761 | 2363 | 1053 | ND |
| curative EC₅₀ (µg mL⁻¹) | 134 | 96 | 76 | ND |

Maximum inhibition of RSV was less pronounced with 54 % but EC₅₀ was the lowest value observed for all viruses with 76 µg mL⁻¹ (Fig. 3C, Tab. 2). No EC₅₀ could be determined for AV as maximum inhibition was 21 % (Fig. 3D). The active control Ribavirin inhibited plaque formation of FluA, RV and RSV by 70-96 % at concentrations between 5-50 µg mL⁻¹. The plant-derived product reduced AV CPE by 44 % at a concentration of 10 µg mL⁻¹.

This study investigated the *in-vitro* antiviral activity of ELOM-080^{®}. The obtained results revealed a strong inhibitory effect against FluA and RVs, moderate efficacy against RSV and minor effects against AV.

Two different approaches of ELOM-080^{®} antiviral activity were tested. First, a preventive approach which encompasses pretreatment of viruses with varying concentrations of ELOM-080^{®} and subsequent assessment of remaining infectivity as a measure of virucidal action. And second, a curative approach in which initially infected cell cultures were treated with ELOM-080^{®} in order to cure the infection. In both settings, ELOM-080^{®} clearly exerted an antiviral activity against respiratory viruses as treatment resulted in lower infectivity and plaques counts in cell cultures. However, the extent to which ELOM-080^{®} treatment decreased viral activity differed significantly between both approaches and in particular between the four tested viruses.

Pretreatment of cell-free viruses with ELOM-080^{®} resulted in maximal reductions of infectivity of FluA, RV and RSV by 79, 54 and 79 %, respectively, which corresponds to comparable efficacy of treatment as observed for the active control SDS. In all three viruses, the treatment effect increased with concentration and incubation time. Whereas, the effect was less pronounced in RV, 24 h incubation in concentration of 2679 µg mL⁻¹ resulted in almost complete disruption of infectivity of FluA and RSV. These effects resemble similar virucidal effects obtained for the monoterpenes β-pinene and limonene when applied against Herpes Simplex Virus (HSV, Astani and Schnitzler, 2014). Reduced HSV infectivity was also observed following pretreatment with eucalyptus oil, α-pinene and 1,8-cineol (Astani et al., 2010). Furthermore, essential oils which comprise the same monoterpenes were found to have antiviral effects against FluA (Vimalanathan and Hudson, 2014). As these or similar components are also integral components of ELOM-080^{®} the virucidal effects against respiratory viruses observed in this study are in agreement with these earlier studies.

In the present study, virucidal effects were most pronounced against the two enveloped viruses FluA and RSV whereas the non-enveloped viruses RV and AV were less affected. It was hypothesized that essential oils interact with the virion envelope or may have masked the structures involved in penetration of cells (Astani and Schnitzler, 2014). Terpenes such as 1,8-cineol are well established penetration enhancers which modify the fluidity of lipid bilayers (Moghimi et al., 2015, Mendanha and Alonso, 2015). As the viral envelope consists of proteins and a lipid bilayer interactions of ELOM-080^{®} with either component are at least possible explanations how pretreatment affects the ability of viruses to attach or penetrate cells and thereby reduce infectivity (Ivanova et al., 2015, Harrison, 2008). Importantly, in the virucidal assay only irreversible changes of e.g. protein structure could have had effects on infectivity as ELOM-080^{®} was not present during the infection itself. Therefore, proteins could have returned to their native state leading to recovery of functionality after the end of pretreatment.

Furthermore, viral genomes are damaged when the capsid integrity is affected by terpenes as observed for treatment of RNA-norovirus with oregano essential oils (Gilling et al., 2014). High ELOM-080^{®} concentrations may have had a similar effect which is supported by the higher sensitivity of the three viruses using less stable RNA compared to the DNA virus.

For the suitability of a medicinal product used for treatment of respiratory diseases, the efficacy in the curative approach is of much higher importance than its sole virucidal activity as it resembles its therapeutic effect in clinical application. In fact, activity of ELOM-080^{®} in the curative assay was an order of magnitude higher in comparison to its preventive action.

Similar to the pretreatment assay, ELOM-080^{®} inhibited AV, the only tested DNA virus, by only about 20 % compared to the solvent control. An absence of treatment effect on AV of eucalyptus' essential oils has already been reported before whereas the mumps virus which is an enveloped RNA-virus was affected (Cermelli et al., 2008). In this study, treatment of infected cells with sinupret served as an internal lab standard did not reach a reduction of 50 % and even established antivirals such as Ribavirin are reported to fail against 5 out of 6 AV species (Morfin et al., 2005).

In contrast to the low sensitivity of the only tested DNA virus AV, ELOM-080^{®} had a clear dose-dependent effect on all three tested RNA viruses. Maximum inhibition was about 80% in FluA and RV and lower in RSV, which however, had lowest EC₅₀. The curative approach covers all phases of the viral life cycle and ELOM-080^{®} treatment may have a comparable therapeutic effect in cell culture as in clinical application. Although applied following infection, cell-free viruses are similarly exposed to the treatment when released from the host cell which potentially results in comparable effects as in the virucidal setting.

Furthermore, pretreatment of cells initially not infected after inoculation with the test substance which may trigger a preventive immune response. Pretreatment of cells lowers baseline concentrations of cytokines and expression of the ICAM-1 receptor to which rhinoviruses attach in order to enter the cytoplasm (Casasnovas and Springer, 1994, Yamaya et al., 2014). Based on results obtained for other monoterpenes, cell pretreatment may to some extent be involved in the antiviral response (Astani et al., 2010, Astani and Schnitzler, 2014). Further research is needed to elucidate whether and how pretreatment with ELOM-080^{®} upregulates cellular defense mechanisms against respiratory viruses.

Attachment of viruses and fusion with the cellular membrane are the first two steps enabling entry of the viral genome into host cells, a process mediated by proteins of the capsid as well as lipids in case of enveloped viruses (Blaas, 2016). Thus, ELOM-080^{®} may disturb entry by modification or masking of surface structures either on the virus itself, as mentioned already above, or on the host cell as hypothesized before (Astani and Schnitzler, 2014). The concentration needed to exert an inhibitory effect in the curative approach was much lower compared to the virucidal assays as proven by high maximal reduction and low EC₅₀ and emphasizes a potential antiviral effect of ELOM-080^{®} in therapeutic treatment. This could result from the presence of ELOM-080^{®} throughout the assay. Therefore not only irreversible but also transient masking and/or modifications of proteins or lipids could affect viral infectivity.

Finally, inhibition of RNA viruses may result from impacts of ELOM-080^{®} on viral replication corresponding to the effect of ribavirin. Astani and coworkers reported reduction of Herpes simplex induced plaques by 30-40% when the monoterpenes 1,8-cineol and α-pinene were added to the assays after infection (Astani et al., 2010). The effects were less pronounced (10-20 %) for β-pinene and limonene in a comparable setting whereas the virus specific DNA-polymerase inhibitor acyclovir lead to 100 % inhibition (Astani and Schnitzler, 2014).

In conclusion, ELOM-080^{®} was shown to have strong antiviral effects against three of the four tested viruses in particular in the curative approach. The test design applied in his study was not intended to specifically determine by which mode of action ELOM-080^{®} inhibits viruses. The volatility of ELOM-080^{®} compounds observed in the cytotoxicity assay may have attenuated effects in the curative assay. Nevertheless, the EC₅₀ values obtained in this study were in a comparable range as levels reported for single monoterpenes which significantly lower FluA viral load and improve survival of infected mice (Li et al., 2016, Lai et al., 2017).

In summary, this study revealed for the first time that ELOM-080^{®} has effects against viral pathogens in *in-vitro* assays which may support its efficacy in clinical treatment of respiratory infections.

### References:

APP, E. M. 2000. Stellenwert der Mukusclearance für das Bronchialsystem-Pathophysiologie und therapeutische Ansätze. In: MEISTER, R. (ed.) Entzündliche Erkrankungen des Bronchialsystems - Ergebnisse der II. Sylter Sekretolyse-Gespräche. Heidelberg: Springer.
ARRUDA, E., PITKÄRANTA, A., WITEK, T., DOYLE, C. A. & HAYDEN, F. G. 1997. Frequency and natural history of rhinovirus infections in adults during autumn. Journal of clinical microbiology, 35, 2864-2868.
ASTANI, A., REICHLING, J. & SCHNITZLER, P. 2010. Comparative study on the antiviral activity of selected monoterpenes derived from essential oils. Phytotherapy Research: An International Journal Devoted to Pharmacological Toxicological Evaluation of Natural Product Derivatives, 24, 673-679.
ASTANI, A. & SCHNITZLER, P. 2014. Antiviral activity of monoterpenes beta-pinene and limonene against herpes simplex virus in vitro. Iranian journal of microbiology, 6, 149.
BAGGA, B., WOODS, C. W., VELDMAN, T. H., GILBERT, A., MANN, A., BALARATNAM, G., LAMBKIN-WILLIAMS, R., OXFORD, J. S., MCCLAIN, M. T. & WILKINSON, T. 2013. Comparing influenza and RSV viral and disease dynamics in experimentally infected adults predicts clinical effectiveness of RSV antivirals. J Antivir Ther, 18, 785-791.
BEGROW, F., BÖCKENHOLT, C., EHMEN, M., WITTIG, T. & VERSPOHL, E. J. 2012. Effect of myrtol standardized and other substances on the respiratory tract: ciliary beat frequency and mucociliary clearance as parameters. Advances in Therapy, 29, 350-358.
BEUSCHER, N., BIEN, E., ELSTNER, E., KIETZMANN, M. & AMON, U. 1997. Myrtol standardized in treatment of sinusitis and bronchitis-Pharmacodynamics and pharmacokinetics. Z Phytotherapie Abstractband, 8, 9-10.
BEUSCHER, N., KIETZMANN, M., BIEN, E. & CHAMPEROUX, P. 1998. Interference of Myrtol standardized with inflammatory and allergic mediators. Arzneimittel-Forschung, 48, 985-989.
BLAAS, D. 2016. Viral entry pathways: the example of common cold viruses. Wiener medizinische Wochenschrift, 166, 211-226.
CASASNOVAS, J. M. & SPRINGER, T. A. 1994. Pathway of rhinovirus disruption by soluble intercellular adhesion molecule 1 (ICAM-1): an intermediate in which ICAM-1 is bound and RNA is released. Journal of virology, 68, 5882-5889.
CERMELLI, C., FABIO, A., FABIO, G. & QUAGLIO, P. 2008. Effect of eucalyptus essential oil on respiratory bacteria and viruses. Current microbiology, 56, 89-92.
DEMURI, G. P., GERN, J. E., MOYER, S. C., LINDSTROM, M. J., LYNCH, S. V. & WALD, E. R. 2016. Clinical features, virus identification, and sinusitis as a complication of upper respiratory tract illness in children ages 4-7 years. The Journal of pediatrics, 171, 133-139. e1.
EHLKEN, B., ANASTASSOPOULOU, A., HAIN, J., SCHRODER, C. & WAHLE, K. 2015. Cost for physician-diagnosed influenza and influenza-like illnesses on primary care level in Germany-results of a database analysis from May 2010 to April 2012. BMC public health, 15, 578.
FOKKENS, W. J., LUND, V. J., MULLOL, J., BACHERT, C., ALOBID, I., BAROODY, F., COHEN, N., CERVIN, A., DOUGLAS, R. & GEVAERT, P. 2012. EPOS 2012: European position paper on rhinosinusitis and nasal polyps 2012. A summary for otorhinolaryngologists. Rhinology, 50, 1-12.
GILLING, D. H., KITAJIMA, M., TORREY, J. & BRIGHT, K. R. 2014. Antiviral efficacy and mechanisms of action of oregano essential oil and its primary component carvacrol against murine norovirus. Journal of applied microbiology, 116, 1149-1163.
GLATTHAAR-SAALMÜLLER, B., MAIR, K. H. & SAALMÜLLER, A. 2017. Antiviral activity of aspirin against RNA viruses of the respiratory tract-an in vitro study. Influenza other respiratory viruses, 11, 85-92.
GRAßMANN, J., HIPPELI, S., DORNISCH, K., ROHNERT, U., BEUSCHER , N. & ELSTNER, E. 2000. Antioxidant Properties of Essential Oils. Possible explanantions for their anti-inflammatory effects. Arzneimittelforschung, 50, 135-139.
HANSEN, M. P., HOFFMANN, T. C., MCCULLOUGH, A. R., VAN DRIEL, M. L. & DEL MAR, C. B. 2015. Antibiotic resistance: what are the opportunities for primary care in alleviating the crisis? Frontiers in public health, 3, 35.
HARRISON, S. C. 2008. Viral membrane fusion. Nature structural molecular biology, 15, 690.
IVANOVA, P. T., MYERS, D. S., MILNE, S. B., MCCLAREN, J. L., THOMAS, P. G. & BROWN, H. A. 2015. Lipid Composition of the Viral Envelope of Three Strains of Influenza Virus□ Not All Viruses Are Created Equal. ACS infectious diseases, 1, 435-442.
JACOBS, S. E., LAMSON, D. M., GEORGE, K. S. & WALSH, T. J. 2013. Human rhinoviruses. Clinical microbiology reviews, 26, 135-162.
KÄRBER, G. 1931. Beitrag zur kollektiven Behandlung pharmakologischer Reihenversuche. Naunyn-Schmiedebergs Archiv für experimentelle pathologie und pharmakologie, 162, 480-483.
KARDOS, P., DINH, Q., FUCHS, K., GILLISSEN, A., KLIMEK, L., KOEHLER, M., SITTER, H. & WORTH, H. 2019. Guidelines of the German Respiratory Society for diagnosis and treatment of adults suffering from acute, subacute and chronic cough. Pneumologie, 73, 143-180.
KASCHKE, O., BEHRBOHM, H. & SYDOW, K. 1997. The influence of a secretolytic drug on mucociliary clearance of the maxillary sinus. Journal of Rhinology, 4, 29-33.
LAI, Y. N., LI, Y., FU, L. C., ZHAO, F., LIU, N., ZHANG, F. X. & XU, P. P. 2017. Combinations of 1, 8-cineol and oseltamivir for the treatment of influenza virus A (H3N2) infection in mice. Journal of medical virology, 89, 1158-1167.
LEMIENGRE, M. B., VAN DRIEL, M. L., MERENSTEIN, D., LIIRA, H., MÄKELÄ, M. & DE SUTTER, A. I. 2018. Antibiotics for acute rhinosinusitis in adults. Cochrane Database of Systematic Reviews*.*
LI, Y., LAI, Y., WANG, Y., LIU, N., ZHANG, F. & XU, P. 2016. 1, 8-Cineol protect against influenza-virus-induced pneumonia in mice. Inflammation, 39, 1582-1593.
MÄKELÄ, M. J., PUHAKKA, T., RUUSKANEN, O., LEINONEN, M., SAIKKU, P., KIMPIMÄKI, M., BLOMQVIST, S., HYYPIÄ, T. & ARSTILA, P. 1998. Viruses and bacteria in the etiology of the common cold. Journal of clinical microbiology, 36, 539-542.
MENDANHA, S. A. & ALONSO, A. 2015. Effects of terpenes on fluidity and lipid extraction in phospholipid membranes. Biophysical chemistry, 198, 45-54.
MOGHIMI, H. R., SHIRAZI, F. H., ARDESTANI, M. S., OGHABIAN, M. A., SAFFARI, M. & SOJOUDI, J. 2015. In vitro and in vivo enhancement of antitumoral activity of liposomal antisense oligonucleotides by cineole as a chemical penetration enhancer. Journal of Nanomaterials, 16, 345.
MORFIN, F., DUPUIS-GIROD, S., MUNDWEILER, S., FALCON, D., CARRINGTON, D., SEDLACEK, P., BIERINGS, M., CETKOVSKY, P., KROES, A. & VAN TOL, M. 2005. In vitro susceptibility of adenovirus to antiviral drugs is species-dependent. Antivir Ther, 10, 225-229.
MOSMANN, T. 1983. Rapid colorimetric assay for cellular growth and survival: application to proliferation and cytotoxicity assays. Journal of immunological methods, 65, 55-63.
PALMENBERG, A. C. & GERN, J. E. 2015. Classification and evolution of human rhinoviruses. Rhinoviruses. Springer.
RANTZSCH, U., VACCA, G., DUCK, R. & GILLISSEN, A. 2009. Anti-inflammatory effects of Myrtol standardized and other essential oils on alveolar macrophages from patients with chronic obstructive pulmonary disease. European Journal of Medical Research, 14, 205-209.
SMITH, S. M., FAHEY, T., SMUCNY, J. & BECKER, L. A. 2017. Antibiotics for acute bronchitis. Cochrane database of systematic reviews*.*
STUCK, B. A., POPERT, U., BEULE, A., JOBST, D., KLIMEK, L., LAUDIEN, M., LELL, M. & VOGL, T. 2017. Rhinosinusitis. Leitlinie der Deutschen Gesellschaft für Hals-Nasen-Ohren-Heilkunde, Kopf- und Hals-Chirurgie.
VIMALANATHAN, S. & HUDSON, J. 2014. Anti-influenza virus activity of essential oils and vapors. American Journal of Essential Oils Natural Products, 2, 47-53.
YAMAYA, M., NISHIMURA, H., NADINE, L. K., OTA, C., KUBO, H. & NAGATOMI, R. 2014. Ambroxol inhibits rhinovirus infection in primary cultures of human tracheal epithelial cells. Archives of pharmacal research, 37, 520-529.

### Example 2

### COVARI study

A randomised, placebo-controlled, double-blind parallel-group trial to assess the efficacy and safety of PB432 (ELOM-080^{®}) in COVID-19 positive hospitalised with acute respiratory insufficiency (ARI).

### Study identifier P2003GF

### BACKGROUND

G. Pohl-Boskamp GmbH & Co. KG ("Pohl-Boskamp") is a family-owned German pharmaceutical company. Pohl-Boskamp plans to carry out a randomised, placebo-controlled, double-blind, parallel-group trial in COVID-19 positive hospitalised in Germany.

The investigational product is a high-dosed GeloMyrtol^{®} forte (ELOM-080^{®}) which should be masked as "PB432" for blinding purposes. The intended indication is atypical or viral pneumonia, in literature sometimes paraphrased as COVID-19 disease. However, the admission diagnosis is often coded as acute respiratory insufficiency (ARI).

### Patient selection and rationale of the study

Eligible hospitalised patients are positive tested of COVID-19 and admitted to a separate isolation ward due to acute respiratory insufficiency (ARI). The clinical picture presents a stable patient with respiratory and/or systemic symptoms or signs at time of admission and a need for supplemental oxygen, but does not present any clinical features suggesting a complicated course of illness (non-ARDS), or need for transfer to intermediate (IMC) or intensive care unit (ICU) [ANZICS 2020, Brogan 2020, Dreher 2020, Gattinoni 2020, Siddiqu 2020, WHO 2020]. The degree of hypoxemia is mild (SpO2 ≤ 94 % and ≤ 92 % for patients with known chronic lung diseases; *alternatively:* 200 mm Hg < PaO₂/FiO₂ ≤ 300 mm Hg) [Ranieri 2012].

### Proposed staging of COVID-19

Typical imaging findings are consistent with early presentation of viral pneumonia (e.g. ground-glass opacification with or without consolidative abnormalities, L-phenotype acc. to preliminary classification [Brogan, 2020, Gattinoni, 2020, Pfeifer 2020]). The patient is able to maintain oxygen saturation above 92% (or above 90% for COPD patients/with chronic lung disease) with up to 5 L/min oxygen via nasal prongs (low flow system via nasal cannula). The degree of hypoxemia is mild (200 mm Hg < PaO₂/FiO₂ ≤ 300 mm Hg at ambient air) [Ranieri 2012].

Patients tested positive for COVID-19 will remain hospitalised until they are symptom-free acc. to medical assessment for at least 48 hours and 2 of their samples are tested negative for SARS-CoV-2 as per RKI's discharge policy [RKI 2020].

### Rates of ICU admission (mechanical ventilation)/progression

Reports suggest that among those who are hospitalised, up to one-quarter need intensive care unit (ICU) admission (China [Yang 2020, Wang 2020, Wu 2020, Guan 2020], Italy [Grasselli 2020a, Livingston 2020], USA [Cheung 2020]).

Profound hypoxemic respiratory failure from acute respiratory distress syndrome (ARDS) is the dominant finding in critically ill patients [Yang 2020, Wang 2020, Huang 2020, Arentz 2020, Bhatraju 2020, Grasselli 2020b, Zhou 2020, Wu 2020b, Chen 2020]. The need for mechanical ventilation in those who are critically ill is high ranging from 42 to 100 percent [Yang 2020, Arentz 2020, Bhatraju 2020, Grasselli 2020b, Wu 2020b]. Other complications included acute kidney injury (and many required renal replacement therapy), elevated liver enzymes and cardiac injury (e.g. cardiomyopathy, pericarditis, pericardial effusion, arrhythmia, and sudden cardiac death) [Yang 2020, Arentz 2020, Goyal 2020, Pfeifer 2020]. Sepsis, shock, and multi-organ failure were less common [Yang 2020, Goyal 2020]. Data on the risk of secondary bacterial pneumonia (hospital-acquired pneumonia) are limited, but it does not appear to be a major feature of COVID-19 [Dreher 2020, Yang 2020]. Only in some cases, a secondary bacterial pneumonia with or without underlying diffuse alveolar damage was found post-mortem [Menter 2020, Wichmann 2020]. Finally, the data have still regarded to be preliminary.

### Respiratory care

Specific aspects of respiratory care relevant to deteriorating patients with COVID-19 before admission to the intensive care unit (ICU) include oxygenation with low flow and high-flow systems, noninvasive ventilation and the administration of nebulised medications. For hospitalised patients who develop progressive symptoms, early admission to the ICU is prudent when feasible.

The World Health Organization (WHO) suggests titrating oxygen to a target peripheral oxygen saturation (SpO₂) of ≥90 percent [WHO 2020]. In general, the lowest possible fraction of inspired oxygen (FiO₂) necessary to meet oxygenation goals, ideally targeting a SpO₂ between 92 and 96 percent, if feasible [Brogan 2020, Brogan 2020b]. This will decrease the likelihood that adverse consequences of supplemental oxygen will develop, such as absorption atelectasis, accentuation of hypercapnia and parenchymal injury [Kamat 2016.]

For patients meeting inclusion for COVID-19 study, supplemental oxygenation with a low flow system via nasal cannula is regarded appropriate (ie, up to 5 L/min). Most cannulae can only provide oxygen at low flow rates - up to 5 litres per minute (L/min) - delivering an oxygen concentration of 28-44%, but usual tables for their conversion are not validated [Pfeifer 2020]. Rates above 5 L/min can result in discomfort to the patient, drying of the nasal passages, and possibly nose bleeds (epistaxis) [Wikipedia 2020].

Higher flows of oxygen may be administered using a simple face mask, Venturi face mask, or nonrebreather mask (e.g. up to 10 to 20 L/minute), but as flow increases, the risk of dispersion also increases, augmenting the contamination of the surrounding environment and staff [Brogan 2020, Pfeifer 2020, Klug 2020]; however, German experts emphasised that using a Venturi face mask is not associated with relevant aerosolization to the environment of a patient [Pfeifer 2020].

### Patients with higher oxygen requirements

When courses progress, higher amounts of oxygen are needed. For non-COVID-19 patients the usual respiratory care options at this point would be high-flow oxygen via nasal cannulae (HFNC) or the initiation of noninvasive ventilation (NIV) [Ranieri 2012]. However, in patients with COVID-19, this decision is controversial and subject to ongoing debate [Cheung 2020, Namendys 2020]. HFNC and NIV are considered aerosol generating procedures with risks of viral transmission including the potential to generate dispersal jets containing droplet or aerosolised viral particles [Hui 2020, Kluge 2020] associated with an increased risk of healthcare worker infection [Raboud 2020]. However, the German society has recently emphasized that healthcare staff can avoid any potential risk of transmission when they use contact and airborne precautions with appropriate personal protection equipment (PPE) [Pfeifer 2020].

Furthermore, many experts advocated the avoidance of both modalities, because of the high likelihood that patients who need these modalities will ultimately, rapidly deteriorate and require mechanical ventilation (e.g. within one to three days) [Brogan 2020, Pfeifer 2020]. For this reason, especially German experts recommended that continuous monitoring with readiness to carry out intubation must be ensured at all times [Pfeifer 2020]. In this context, German experts emphasized, that in contrary to Germany those fatal courses were mainly observed in Italy and New York in the beginning, when critical care resources were overloaded and patients already in an advanced stage and with signs of rapid progression before [Lenzen-Schulte, 2020].

Considering these noninvasive modalities demand elaborate precautions (i.e. airborne infection isolation room, full personal protective equipment) [ANZICS 2020]. Therefore, experts strongly recommend not use high-flow nasal oxygen (HFNC) therapy for patients with hypoxemia associated with COVID-19 in shared wards [ANZICS 2020].

Mucolytics have been proven to reduce sputum viscosity and have also been shown to modulate airway inflammation by reducing the cytokine release and subsequent respiratory tract tissue damage [Khomich 2018; (for Myrtol) Begrow 2012, Beuscher 1998, Graßmann 2000, Kaschke 1997, Lai 2014, Rantzsch 2009]. Experts suggested that consideration should be given to empirically add e.g. Carbocisteine [Imraan 2020] or *N*-acetylcysteine [Anon 2020] to the current treatment regimen in patients infected with COVID-19. Unfortunately, they are considered as nebulised therapy. However, nebulizers are associated with aerosolization and potentially increase the risk of virus transmission [ANZICS 2020]. In April 2020, some experts recommended that nebulised therapy should generally be avoided [Brogan 2020], but in their update May 2020 the same experts deleted the whole chapter on "nebulized medications" [Brogan 2020b]. Acc. to WHO there is insufficient evidence to classify nebuliser therapy as an aerosol-generating procedure that is associated with transmission of COVID-19 and called for more research [WHO 2020].

### PB432

Enteric coated capsules containing 300 mg of PB432 (trade name in Germany: GeloMyrtol^{®} forte) are well established in the treatment of acute and chronic inflammatory diseases of the respiratory tract, bronchitis and sinusitis in particular, and are approved in many European and non-European countries.

The efficacy, safety and tolerability of PB432 was investigated in well-designed, prospective, large scale, multi-centre, randomised, double-blind, placebo- and actively controlled, parallel-group trials; all trials were planned, conducted and reported according to the guidelines of "Good Clinical Practice". The current documentation constitutes the best available external evidence for a clinically relevant therapeutic benefit of the active ingredient in the treatment of acute and chronic inflammatory respiratory diseases, bronchitis and sinusitis, in particular. PB432 is recommended in several German and international treatment guidelines, independent organisations like Cochrane Collaboration reviewed clinical studies with a positive vote [@].

### Pharmacodynamics

PB432 displays a broad spectrum of pharmacological properties and its efficacy can be explained by its secretomucolytic, secretomotoric, and mucociliary clearance enhancing capabilities [Begrow 2012, Cao 2010, Kaschke 1997].

A restored mucociliary clearance contributes to the improvement of the volume conductivity in the respiratory tract. Investigations on the effect of PB432 on *in vivo* mucociliary clearance in mice and ciliary beat frequency (CBF) in rat tracheal rings showed that PB432 accelerated both CBF and mucociliary transport in a concentration-dependent manner [Begrow 2012]. In an air-liquid interface model with primary human sinonasal epithelial cells basolateral application of PB432 stimulated both chloride efflux and cilia beat frequency resulting in a synergistic effect dramatically augmenting mucociliary transport velocity, which supports the clinical efficacy of PB432 in respiratory inflammatory disorders [Lai 2014].

In addition, PB432 has been shown to exert antioxidant properties [Graßmann 2000] and anti-inflammatory actions [Beuscher 1998, Rantzsch 2009]. For 1.8-cineole, one of the main monoterpenes and key biological marker substance of PB432, inhibition of mediator release in LPS-stimulated monocytes isolated from patients suffering from chronic obstructive pulmonary disease (COPD) or healthy subjects has been shown [Juergens 2004]. The anti-inflammatory properties of PB432/1,8-cineol have been shown in animal models, where airway inflammatory parameters were reduced or absent in 1,8-cineol treated OVA-sensitized guinea pigs, with reduced levels of TNF-α, IL-1β in BALF [Bastos 2011] and in clinical trials on asthma, bronchitis, or COPD [Juergens 2003, Worth 2009, Fischer 2013]. In a mouse model of acute lung injury PB432 attenuated lipopolysaccharide (LPS)-induced lung injury by reducing neutrophil counts and levels of inflammatory cytokines [Yu 2016]. Furthermore, in a mouse model of radiation-induced lung injury PB432 in contrast to prednisone reduced lung coefficient and collagen deposition, and inhibited the release of pro-inflammatory cytokines [Zhao 2016].

Recently, the potential anti-viral properties of PB432 were investigated. Therefore, *in-vitro* studies were conducted to determine the effect of PB432 on 4 important viral pathogens of respiratory infections: Influenza A (FluA), Rhinovirus (RV), Respiratory Syncytial Virus (RSV) and Adenovirus (AV) (manuscript in preparation). The obtained results revealed a virucidal effect of more than 50% inhibition of viral infectivity for FluA, RV and RSV following pretreatment of viruses prior to infection. In a second, curative approach EC₅₀ values for the three RNA viruses tested (FluA, RV, RSV) were even an order of magnitude lower compared to results for the virucidal effect which emphasize a potential role in clinical therapy. PB432 had a clear dose-dependent effect on all three tested RNA viruses, presumably resulting from a potential effect on integrity and functionality of viral RNA, indicating efficacy also for the treatment of diseases caused by viruses belonging to the Coronaviridae with a plus sense single stranded RNA genome.

### Supporting strategies for hypoxemia

In hospitalised patients with acute exacerbations of COPD (AECOPD) PB432 demonstrated in a phase IV trial improvement of oxygen saturation versus placebo [Ulmer & Schött 1991]. Twenty inpatients with AECOPD, aged on average 62 years (mean; range 38 to 80 years), were allocated to an oral treatment of 4 x 300 mg PB432/day versus matched placebo for 14 days. All patients were treated with corticosteroids and beta(2)-agonists; no patient received supplemental oxygen.

At time of admission the partial pressure of oxygen was 62.8 ± 6.5 mmHg in the PB432 group and 66.8 ± 9.0 mmHg in the placebo group measured in an arterial blood gas (ABG) test. The oxygen saturation (approximated acc. to Severinghaus) was 92 % ±2 % in the PB432 group and 93 % ± 3 % in the placebo group at treatment start. The P/F ratio (Horowitz index; FiO₂ 21%, ambient air) was 299 ± 31 mmHg and 318 ± 43 mmHg (PB432, resp. Placebo). These findings were consistent with a *mild* oxygenation impairment.

During the clinical course the partial pressure of oxygen increased by +10.8 % ± 3.3 % versus +7.5 % ± 3.2 % (Mean ±SEM; n= 20; PB432 resp. Placebo) which might indicate that PB432 improves oxygen saturation (**Fig.1**). The latter effect supports strategies for hypoxemia (warrants a lowest possible fraction of inspired oxygen (FiO₂) and helps to prevent progression/deterioration and/or proceeding to HFNC/NIV modalities (on IMC) or even early intubation (on ICU) if the patient's course escalates beyond 6 L/min with continued hypoxemia or increased work of breathing).

Given the essential role of inflammation and viral load within the course of COVID-19, treatment of these patients with high-dose PB432 is expected to result not only in the improvement of oxygen saturation but also in the decrease of viral load and inflammatory parameters.

It is therefore expected that a double-dosed daily treatment with 4 x 600 mg PB432 per os results in a pronounced effect on the mentioned outcome parameters. Alternatively, also a treatment with 4 x 300 mg is feasible.

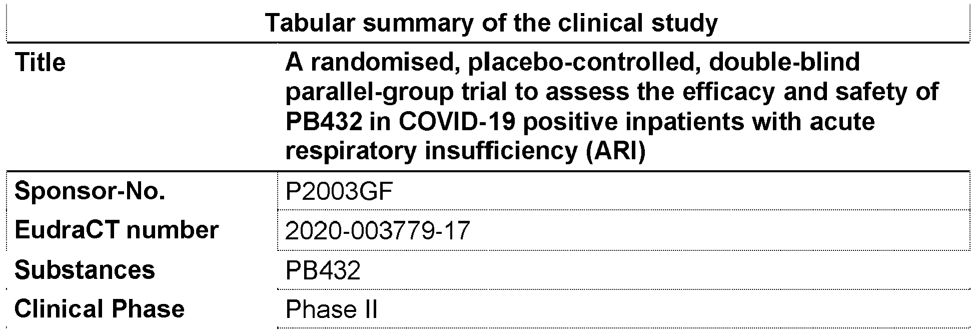

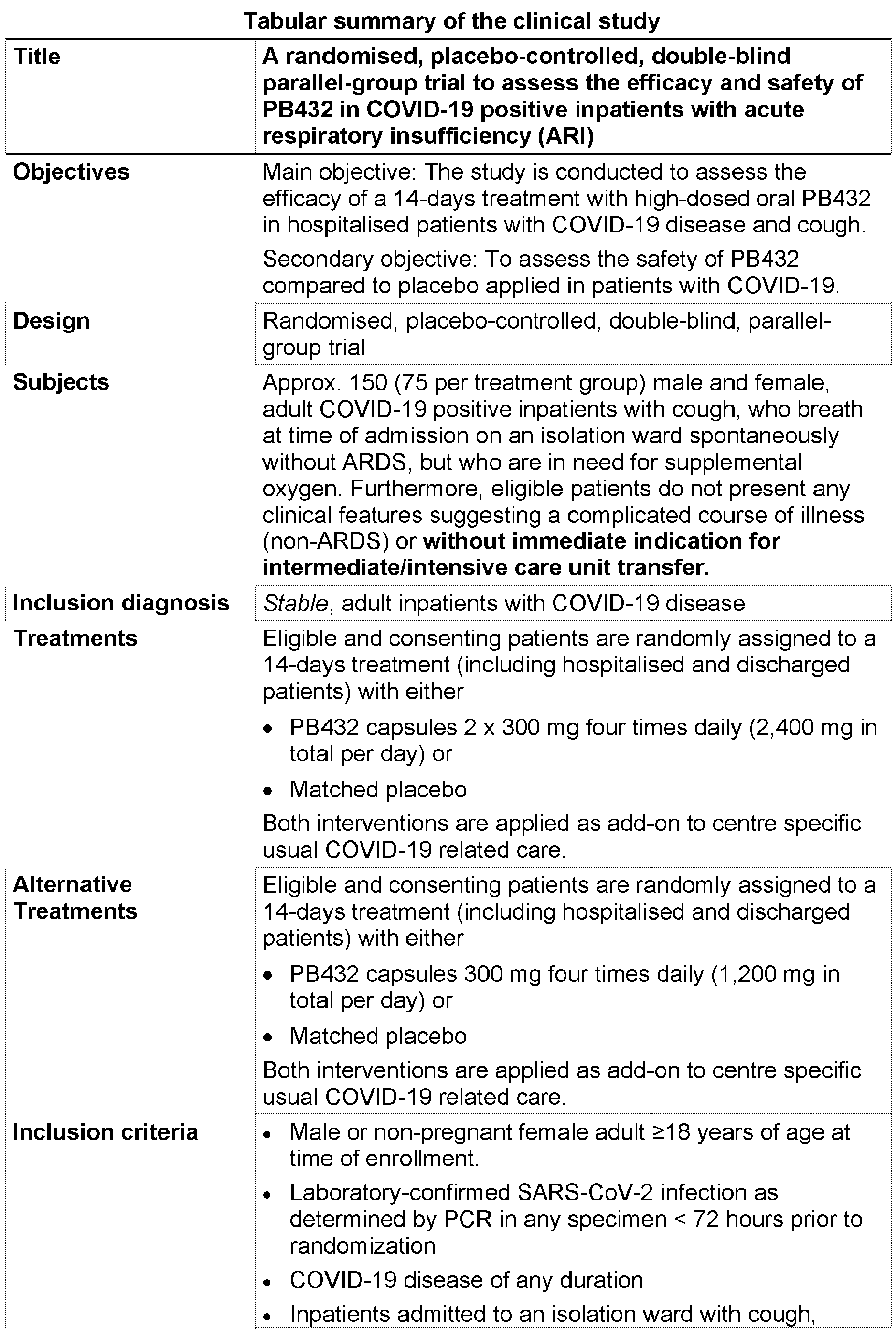

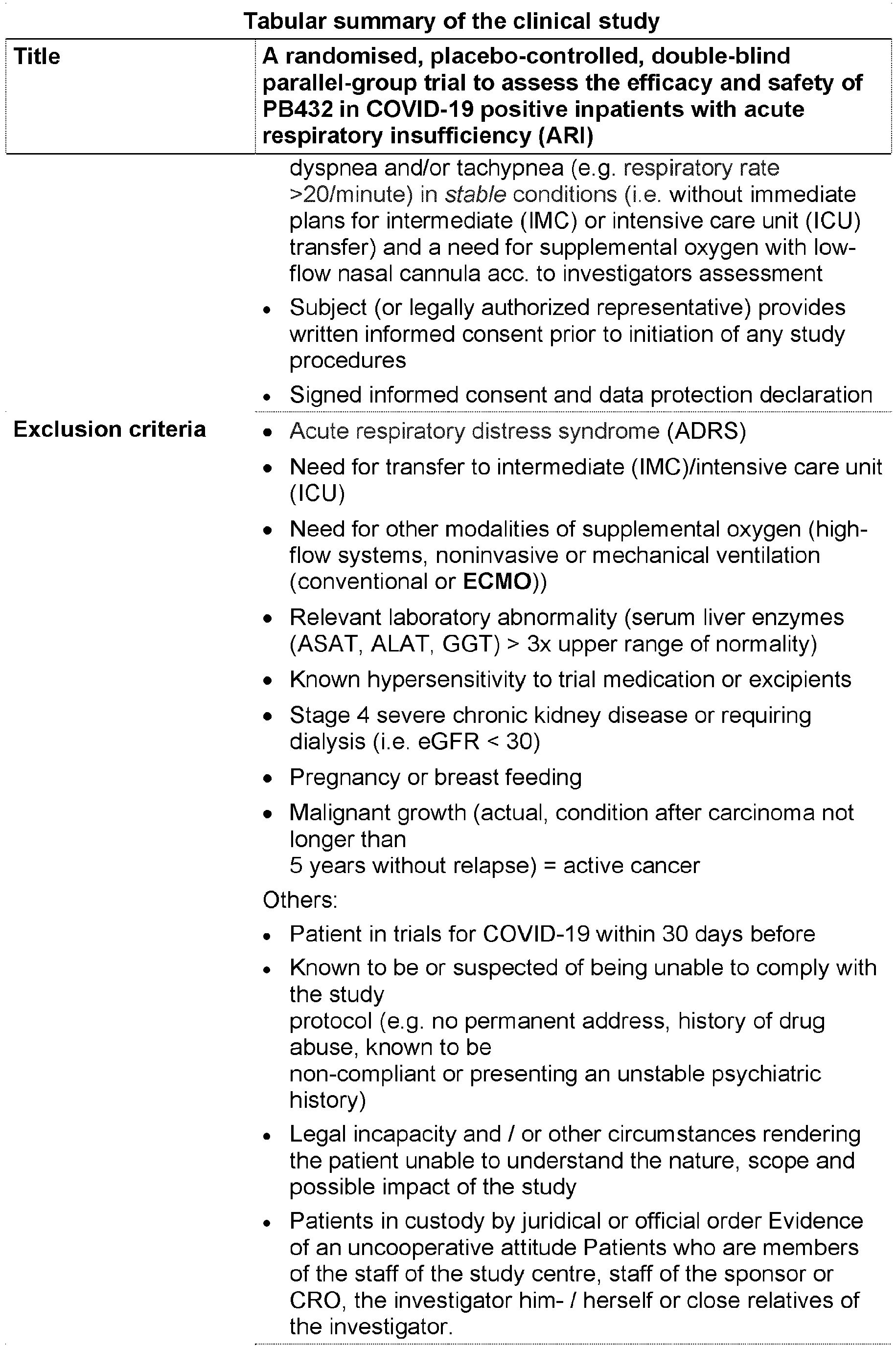

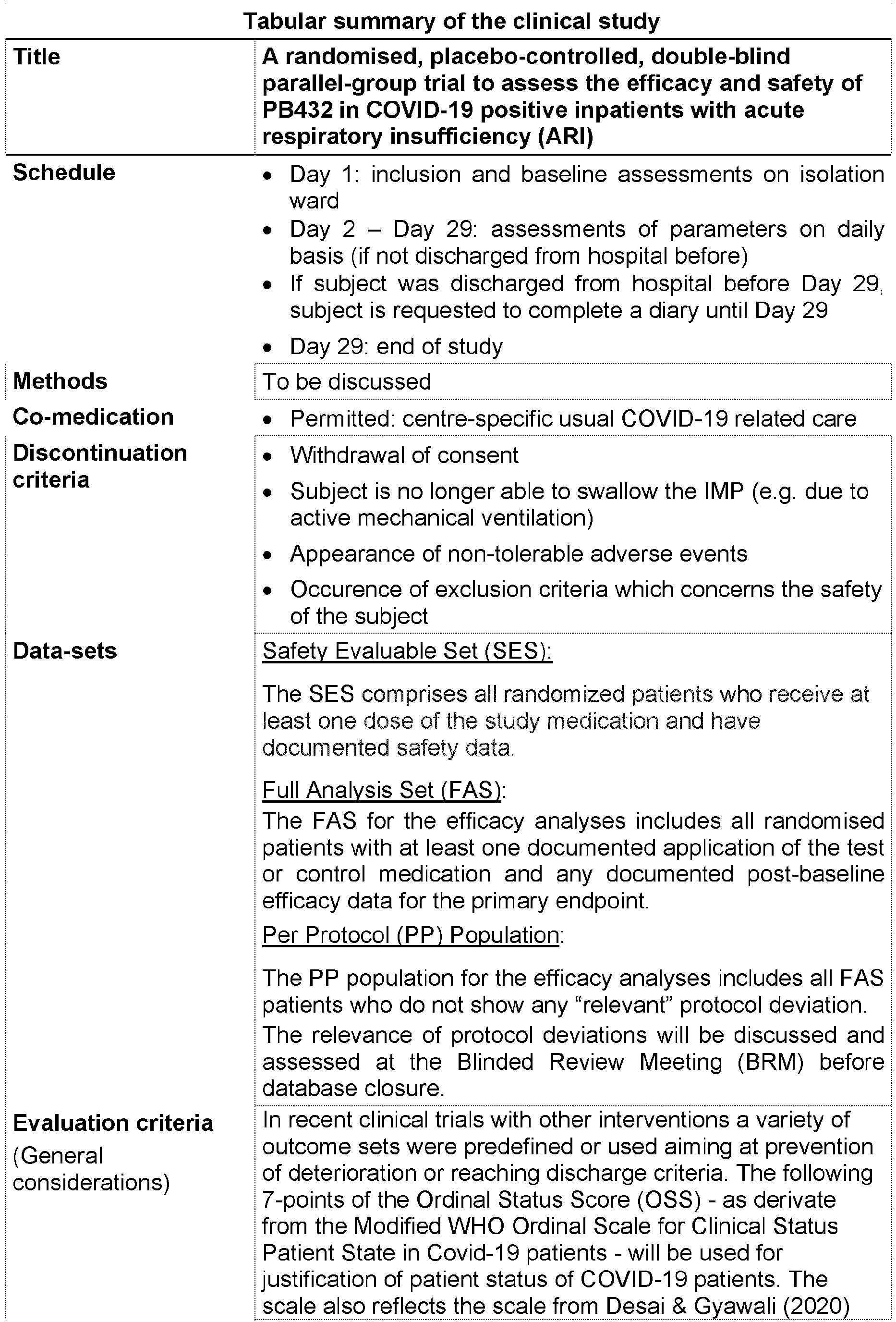

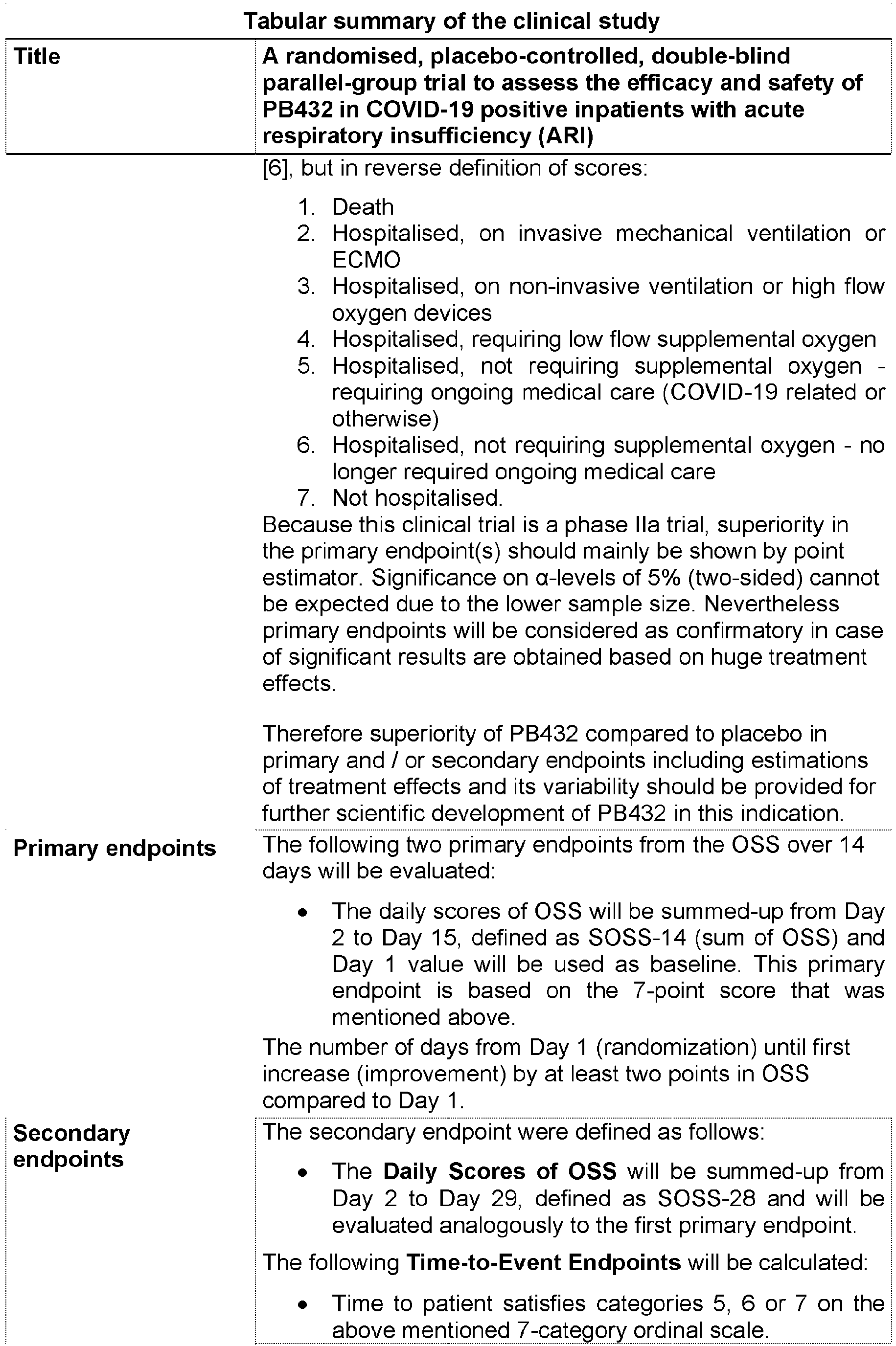

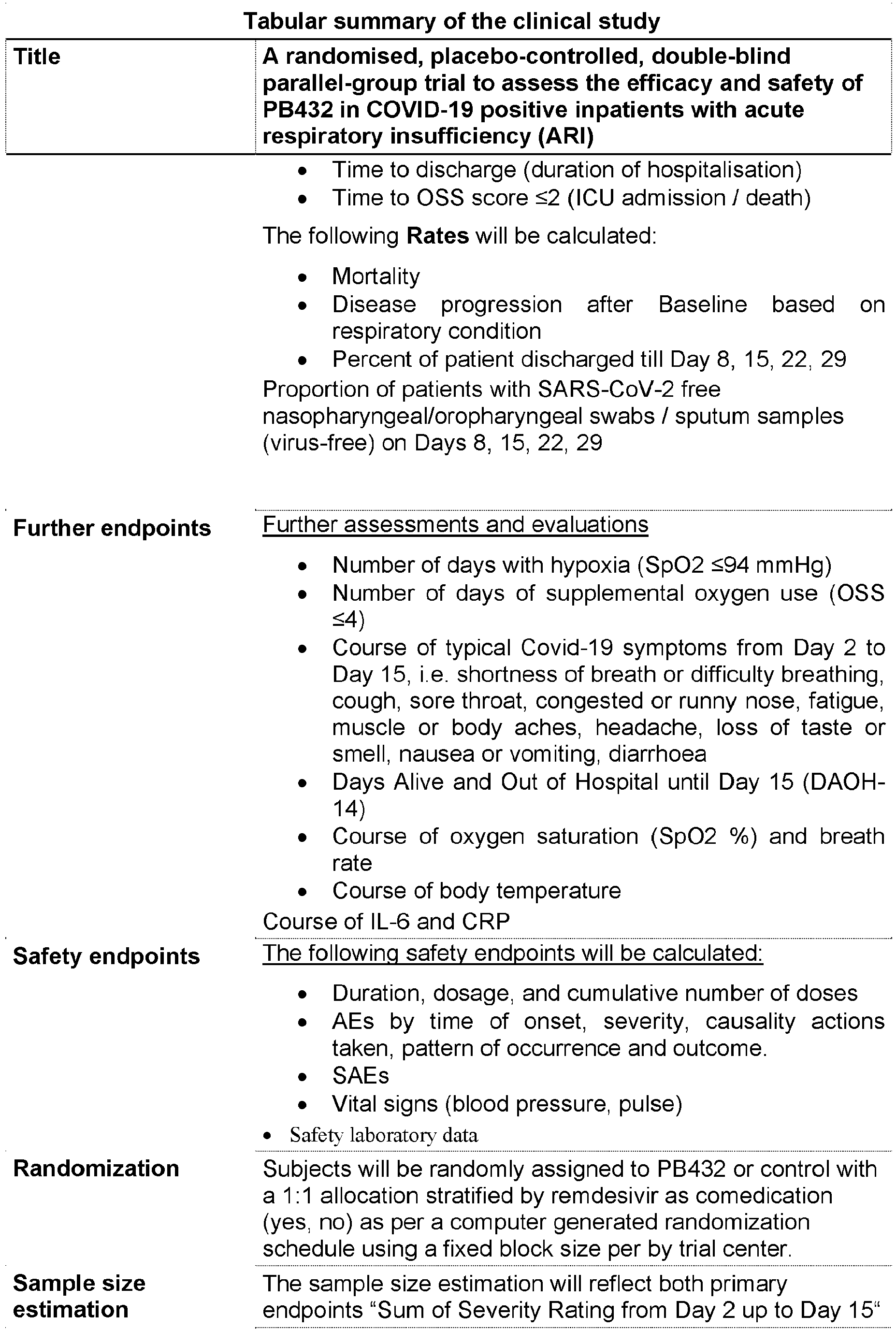

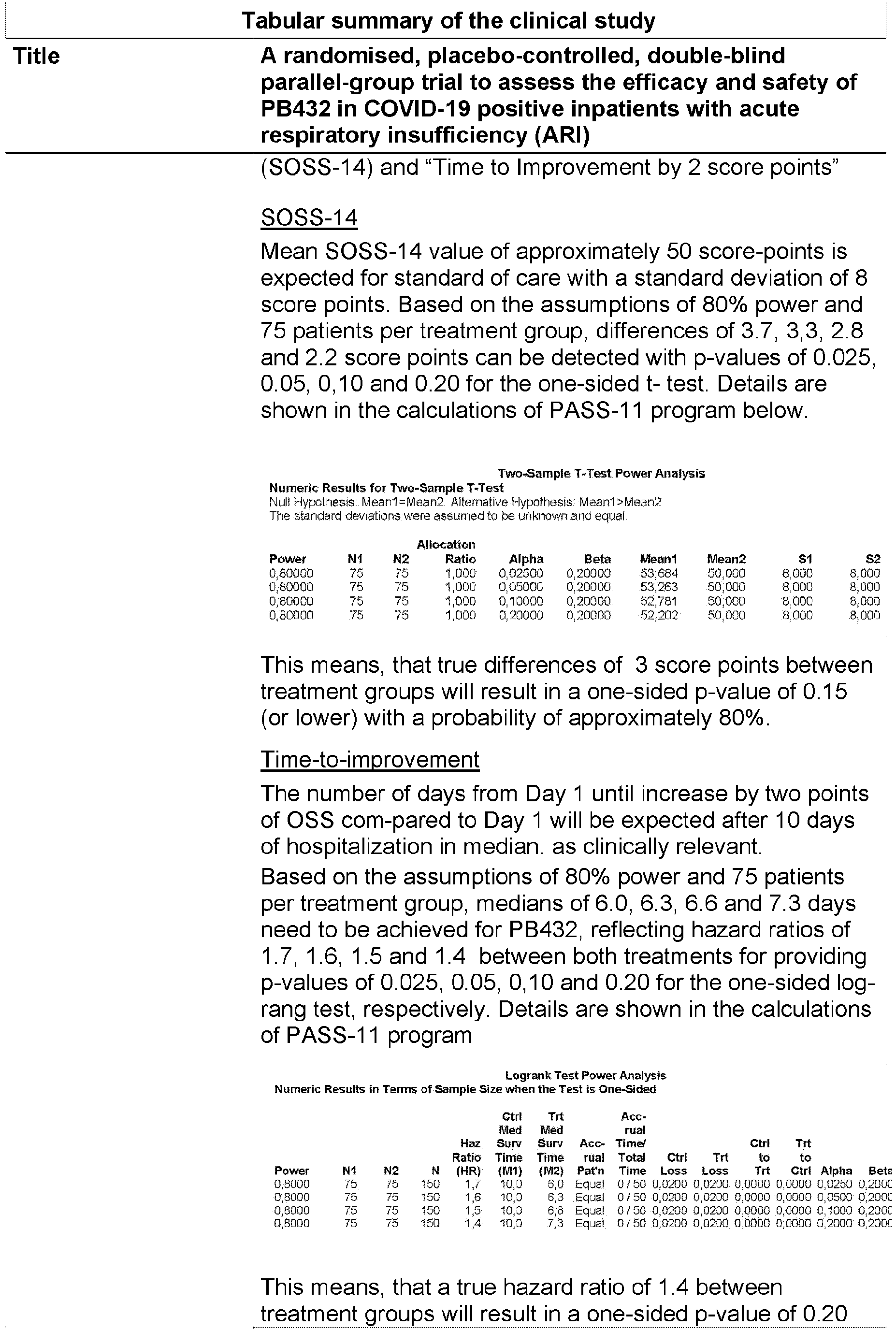

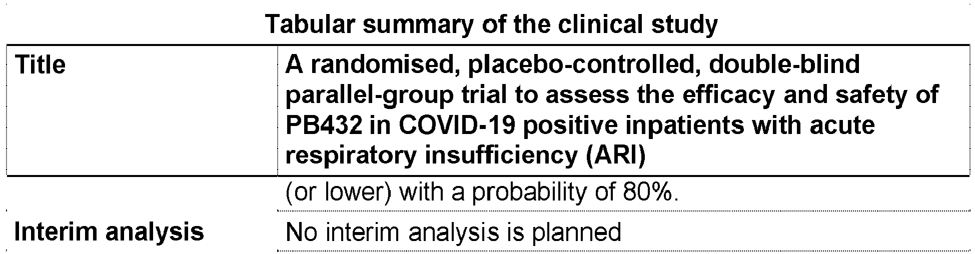

### GCP-adherence, Regulatory Requirements

All study relevant procedures should adhere to the principles of the pertinent Declaration of Helsinki. All study relevant procedures should comply with the pertinent GCP-principles, German Drug Law and other national regulations; sufficient and adequate provisions for the orderly quality management of the trial should be provided for and adherence with these provisions should be closely monitored.

### References

Anonymous. Treatment regimens of COVID-19 (Russian: Tepa 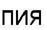 COVID-19). Russian Federal Medical-Biological Agency. April 2020
Arentz M, Yim E, Klaff L, et al. Characteristics and Outcomes of 21 Critically III Patients With COVID-19 in Washington State. JAMA 2020
Australian National COVID-19 Clinical Evidence Taskforce - National COVID-19 Clinical Evidence Taskforce. Australian guidelines for the clinical care of people with COVID-19. Version 2.1 - 4/23/20. https://app.magicapp.org/app#/quideline/4179
Bastos, V. P. et al. Inhaled 1, 8-Cineole Reduces Inflammatory Parameters in Airways of Ovalbumin-Challenged Guinea Pigs. Basic & clinical pharmacology & toxicology 108, 34-39 (2011)
Beigel JH. Remdesivir for the Treatment of Covid-19 - Preliminary Report. Published on May 22, 2020, at NEJM.org. DOI: 10.1056/NEJMoa2007764. https://www.neim.org/doi/full/10.1056/NEJMoa2007764
Begrow F, Böckenholdt C, Ehmen M, Wittig Th, Verspohl EJ. Effects of Myrtol Standardized and Other Substances on the Respiratory Tract: Ciliary Beat Frequency and Mucociliary Clearance as Parameters. Adv Ther (2012) 29(4):350-358
Beuscher N, Kietzmann M, Bien E et al. Interference of Myrtol standardized with inflammatory and allergic mediators. Arzneim.-Forsch./Drug Res. 1998; 48; 985-989
Bhatraju PK, Ghassemieh BJ, Nichols M, et al. Covid-19 in Critically III Patients in the Seattle Region - Case Series. N Engl J Med 2020
Brogan G, Campbell N, Durie M, Nickson C. Coronavirus disease 2019 (COVID-19). LITFL Critical Care Compendium. Last update 19-April-2020. https://litfl.com/coronavirus-disease-2019-covid-19/
Brogan G, Campbell N, Durie M, Nickson C. [Brogan 2020b]. Coronavirus disease 2019 (COVID-19). LITFL Critical Care Compendium. Last update 30-May-2020. https://litfl.com/coronavirus-disease-2019-covid-19/#19-management-oxygenation-and-ventilation
Cao, L. et al. Effect of Myrtol standardized on mucus hypersecretion and clearance of Pseudomonas aeruginosa in a rat model of chronic obstructive pulmonary disease. Arzneimittel-Forschung 61, 685-692 (2010)
CDC COVID-19 Response Team. Severe Outcomes Among Patients with Coronavirus Disease 2019 (COVID-19) - United States, February 12-March 16, 2020. MMWR 2020; 69(12), 343-346. [https://www.cdc.gov/mmwr/volumes/69/wr/pdfs/mm6912e2-H.pdf]
Chen N, Zhou M, Dong X, et al. Epidemiological and clinical characteristics of 99 cases of 2019 novel coronavirus pneumonia in Wuhan, China: a descriptive study. Lancet 2020; 395:507
Cheung JC, Ho LT, Cheng JV, et al. Staff safety during emergency airway management for COVID-19 in Hong Kong. Lancet Respir Med 2020; 8:e19
Desai A and Gyawali B. Endpoints used in phase III randomized controlled trials of treatment options for COVID-19, EClinical Medicine (2020), https://doi.org/10.1016/j.eclinm.2020.100403.
Dreher M, Kersten A, Bickenbach J, Balfanz P, Hartmann B, Cornelissen C, Daher A, Stöhr R, Kleines M, Lemmen SW, Brokmann JC, Müller T, Müller-Wieland D, Marx G, Marx N: The characteristics of 50 hospitalized COVID-19 patients with and without ARDS. Dtsch Arztebl Int 2020; 117: 271-8
Fischer, J. & Dethlefsen, U. Efficacy of cineole in patients suffering from acute bronchitis: a placebo-controlled double-blind trial. Cough 9, 25 (2013)
Gattinoni L. et al. COVID-19 pneumonia: different respiratory treatment for different phenotypes? (2020) Intensive Care Medicine; DOI: 10.1007/s00134-020-06033-2
Goyal P, Choi JJ, Pinheiro LC, et al. Clinical Characteristics of Covid-19 in New York City. N Engl J Med 2020
Grasselli G, Pesenti A, Cecconi M. Critical Care Utilization for the COVID-19 Outbreak in Lombardy, Italy: Early Experience and Forecast During an Emergency Response. JAMA 2020
Grasselli G, Zangrillo A, Zanella A, et al. Baseline Characteristics and Outcomes of 1591 Patients Infected With SARS-CoV-2 Admitted to ICUs of the Lombardy Region, Italy. JAMA 2020
Graßmann J, Hippeli S, Dornisch K et al. Antioxidant Properties of Essential Oils. Arzneimittelforschung 2000; 50:135-139
Grein J et al., Compassionate Use of Remdesivir for Patients with Severe Covid-19. N Engl J Med 2020;382:2327-36. DOI: 10.1056/NEJMoa2007016
Guan WJ, Ni ZY, Hu Y, et al. Clinical Characteristics of Coronavirus Disease 2019 in China. N Engl J Med 2020
Huang C, Wang Y, Li X, et al. Clinical features of patients infected with 2019 novel coronavirus in Wuhan, China. Lancet 2020; 395:497
Hui DS, Chow BK, Thomas Lo, Owen TV, Tsang FW. Ko, SS, Ng T, Gin M, Chan TV. Exhaled air dispersion during high-flow nasal cannula therapy versus CPAP via different masks. European Respiratory Journal 2019; 53(4). https://doi.org/10.1183/13993003.02339-2018
Imraan F. Jhetam. Rapid response to Vetter P. Covid-19: a puzzle with many missing pieces. BMJ 2020;368:m627
Juergens, U. R. et al. Inhibitory activity of 1, 8-cineol (eucalyptol) on cytokine production in cultured human lymphocytes and monocytes. Pulmonary Pharmacology & Therapeutics 17, 281-287 (2004)
Juergens, U. et al. Anti-inflammatory activity of 1.8-cineol (eucalyptol) in bronchial asthma: a double-blind placebo-controlled trial. Respiratory Medicine 97, 250-256 (2003
Kamat SS. Practical Applications of Mechanical Ventilation. Jaypee Brothers Medical Publishers Ltd. 2nd Edition 2016. p 232
Kaschke O, Behrbohm H, Sydow K. The influence of a secretolytic drug on mucociliary clearance of the maxillary sinus. J Rhinol. 1997; 4(1): 29-33
Khomich O, Kochetkov S, Bartosch B, et al. Redox Biology of Respiratory Viral Infections. Viruses 2018;10:392
Kluge S, Janssens U, Welte T, Weber-Carstens S, Marx G, Karagiannidis C:[Recommendations for critically ill patients with COVID-19]. Med Klin Intensivmed Notfmed 2020. doi: 10.1007/s00063-020-00674-3 (Epub ahead of print)
Lai Y, Dilidaer D, Chen B, Xu G, Shi J, Lee RJ, Cohen NA. In vitro studies of Myrtol on upper airway physiology pertaining to mucociliary clearance. American Journal of Rhinology & Allergy, 2014; 28 (3): 244-248
Lenzen-Schulte M. Richtig beatmen bei COVID-19. Dtsch Arztebl 2020; 117(18): A938
Livingston E, Bucher K. Coronavirus Disease 2019 (COVID-19) in Italy. JAMA 2020
Menter T, Haslbauer JD, Nienhold R, et al. Post-mortem examination of COVID19 patients reveals diffuse alveolar damage with severe capillary congestion and variegated findings of lungs and other organs suggesting vascular dysfunction [published online ahead of print, 2020 May 4]. Histopathology. 2020. https://pubmed.ncbi.nlm.nih.gov/32364264/
Namendys-Silva SA. Respiratory support for patients with COVID-19 infection. Lancet Respir Med 2020; 8:e18
Peng-Jiu Yu, Li-Mei Wan, Shan-He Wan, Wen-Ying Chen, Hui Xie, Dong-Mei Meng, Jia-Jie Zhang & Xiang-Lin Xiao (2016) Standardized myrtol attenuates lipopolysaccharide induced acute lung injury in mice, Pharmaceutical Biology, 54:12, 3211-3216
Pfeifer M, Ewig S, Voshaar T, Randerath W, Bauer T, Geiseler J, Dellweg D, Westhoff M, Windisch W, Schönhofer B, Kluge S, Lepper Ph. Positionspapier zur praktischen Umsetzung der apparativen Differenzialtherapie der akuten respiratorischen Insuffizienz bei COVID-19. 17. April 2020. https://pneumologie.de/fileadmin/user upload/COVID-19/20200417 DGP app. Differenzialtherapie ARI COVID-19.pdf
Raboud J, Shigayeva A, McGeer A, Bontovics E, Chapman M, Gravel D, et al. (2010) Risk Factors for SARS Transmission from Patients Requiring Intubation: A Multicentre Investigation in Toronto, Canada. PLoS ONE 5(5): e10717
Ranieri VM, Rubenfeld GD, Thompson BT, et al.: Acute respiratory distress syndrome: the Berlin Definition. Jama 2012; 307: 2526-33
Rantzsch G, Vacca R, Dück R, Gillissen A. Anti-inflammatory effects of Myrtol standardized and other essential oils in alveolar macrophages from patients with chronic obstructive pulmonary disease. Eur J Med Res 2009; 14: 205-209
Robert Koch Institute. COVID-19. Hospital discharge management [German: COVID-19: Kriterien zur Entlassung aus dem Krankenhaus bzw. aus der häuslichen Isolierung. https://www.rki.de/DE/Content/InfAZ/N/Neuartiges Coronavirus/Entlassmanagem ent.html#doc13671260bodyText1 [24-April-2020]
Robert Koch Institute - Ständiger Arbeitskreis der Kompetenz- und Behandlungszentren für Krankheiten durch hochpathogene Erreger (STAKOB). Hinweise zu Erkennung, Diagnostik und Therapie von Patienten mit COVID-19 [17-April-2020]
Schinko, H., Funk, G., Meschkat, M. et al. Arterielle Blutgasanalyse. Wien. Klin. Wochenschr. Educ 12, 115-130 (2017)
Simonds AK, Hanak A, Chatwin M et al. Evaluation of droplet dispersion during non-invasive ventilation, oxygen therapy, nebuliser treatment and chest physiotherapy in clinical practice: implications for
management of pandemic influenza and other airborne infections. Health Technol Assess 2010; 14: 131-172
Siddiqu HK, Mehra MR. COVID-19 Illness in Native and Immunosuppressed States: A Clinical-Therapeutic Staging Proposal. Journal of Heart and Lung Transplantation. doi: 10.1016/j.healun.2020.03.012
Ulmer WT, Nolte D, Lecheler J. Schafer Th. Die Lungenfunktion. Methodik und klinische Anwendung. 7. Auflage 2003. Thieme Verlagsgruppe, Stuttgart, New York, Delhi, Rio
Ulmer W. T., Schött D.: Chronic obstructive Bronchitis. Effect of GeloMyrtol forte in a placebo-controlled double-blind study. Fortschritte der Medizin 1991, 109(27), 547-550 [English translation]
Wang D, Hu B, Hu C, et al. Clinical Characteristics of 138 Hospitalized Patients With 2019 Novel Coronavirus-Infected Pneumonia in Wuhan, China. JAMA 2020
Wang Y et al. Remdesivir in Adults with Severe COVID-19: A Randomised, Double-Blind, Placebo-Controlled, Multicentre Trial. Lancet 2020. https://www.thelancet.com/action/showPdf?pii=S0140-6736%2820%2931022-9
WHO (2020) Clinical management of severe acute respiratory infection when novel coronavirus (2019-nCoV) infection is suspected Interim guidance. 13-March 2020. https://www.who.int/docs/default-source/coronaviruse/clinical-management-of-novel-cov.pdf
Wichmann D, Sperhake JP, Lütgehetmann M, et al. Autopsy Findings and Venous Thromboembolism in Patients With COVID-19 [published online ahead of print, 2020 May 6]. Ann Intern Med. 2020;10.7326/M20-2003. https://www.acpjournals.org/doi/10.7326/M20-2003
Wikipedia. Nasal cannula. https://en.wikipedia.org/wiki/Nasal cannula [24.04.2020]
Worth, H., Schacher, C. & Dethlefsen, U. Concomitant therapy with Cineole (Eucalyptole) reduces exacerbations in COPD: a placebo-controlled double-blind trial. Respir Res 10, 69 (2009)
Wu C, Chen X, Cai Y, et al. Risk Factors Associated With Acute Respiratory Distress Syndrome and Death in Patients With Coronavirus Disease 2019 Pneumonia in Wuhan, China. JAMA Intern Med 2020
Wu Z, McGoogan JM. Characteristics of and Important Lessons From the Coronavirus Disease 2019 (COVID-19) Outbreak in China: Summary of a Report of 72 314 Cases From the Chinese Center for Disease Control and Prevention. JAMA 2020
Yang X, Yu Y, Xu J, at al. Clinical course and outcomes of critically ill patients with SARS-CoV-2 pneumonia in Wuhan, China: a single-centered, retrospective, observational study. Lancet 2020
Yung-Fang Tu et al. A Review of SARS-CoV-2 and the Ongoing Clinical Trial. International Journal of Molecular Sciences. 2020
Zhao DY, Hong-jin Qu Jia-ming Guo Hai-nan Zhao Yan-yong Yang Pei Zhang Kun Cao Xiao Lei Jian-guo Cui Cong Liu Jian-ming Cai Fu Gao Bai-long Li. Protective Effects of Myrtol Standardized Against Radiation-Induced Lung Injury. Cell. Physiol. Biochem. 2016;38:619-634
Zhou F, Yu T, Du R, et al. Clinical course and risk factors for mortality of adult inpatients with COVID-19 in Wuhan, China: a retrospective cohort study. Lancet 2020; 395:1054.

## Claims

1. A composition comprising eucalyptus oil and sweet orange oil for use in a method for the treatment of a viral infection.

2. The composition for use according to claim 1, wherein the composition comprises eucalyptus oil, sweet orange oil, myrtle oil and lemon oil.

3. The composition for use according to any of claims 1 or 2, wherein the composition is ELOM-080^{®}.

4. The composition for use according to any of claims 1 to 3, wherein the virus is selected from the group consisting of enveloped viruses or non-enveloped viruses.

5. The composition for use according to claim 4, wherein the virus is an enveloped virus.

6. The composition for use according to claim 5, wherein the virus is an RNA virus, preferably selected from the group consisting of influenza virus, preferably influenza A virus, RSV, a corona virus, HIV, parainfluenzavirus.

7. The composition according to claim 6, wherein the corona virus is SARS-Cov-2.

8. The composition for use according to claim 4, wherein the virus is a non-enveloped virus.

9. The composition for use according to claim 8, wherein the virus is an RNA virus, preferably selected from the group consisting of HRV.

10. The composition for use according to any of claims 1 to 9, wherein the infection causes hypoxemia or an acute respiratory distress syndrome.

11. The composition for use according to any of claims 1 to 10, including the treatment of a viral disease caused by the viral infection.

12. The composition for use according to claim 11, wherein the viral disease is an acute or chronic viral respiratory disease, preferably a viral pneumonia, a viral sinusitis and rhinosinusitis or a viral bronchitis.

13. The composition for use according to claim 12, wherein the viral respiratory disease is COVID-19.
